# EUROPEAN PATENT SPECIFICATION

(11) **EP 1 611 115 B1**
(45) Date of publication and mention of the grant of the patent: **22.08.2012**
(21) Application number: 04720788.1
(22) Date of filing: 15.03.2004
(51) Int. Cl.: C07D 277/66, A61K 31/428, A61P 25/28

(54) **BENZOTHIAZOLE DERIVATIVE COMPOUNDS, COMPOSITIONS AND USES**
BENZOTHIAZOLDERIVAT-VERBINDUNGEN, ZUSAMMENSETZUNGEN UND VERWENDUNG
COMPOSES DERIVES DE BENZOTHIAZOLE, COMPOSITIONS ET UTILISATIONS

(30) Priority: 14.03.2003 US 388173; 22.08.2003 US 645847
(43) Date of publication of application: 04.01.2006
(73) Proprietor: University of Pittsburgh - of the Commonwealth System of Higher Education, Pittsburgh, PA 15620-4002 (US)
(72) Inventor: KLUNK, William, E., Pittsburgh, Pennsylvania 15218 (US); MATHIS, JR., Chester A., Pittsburgh, Pennsylvania 15238 (US); WANG, Yanming, Dr., Beachwood OH 44122 (US)
(74) Representative: Plougmann & Vingtoft A/S
(86) International application number: PCT/US2004/007797
(87) International publication number: WO 2004/083195

(56) References cited:
- WO-A-01/14354
- WO-A-02/16333
- WO-A-02/085903
- US-A1- 2003 236 391
- WANG Y ET AL: "SYNTHESIS AND EVALUATION OF 2-(3'-IODO-4'-AMINOPHENYL)-6-HYDROXYBENZO THIAZOLE FOR IN VIVO QUANTITATION OF AMYLOID DEPOSITS IN ALZHEIMER'S DISEASE" JOURNAL OF MOLECULAR NEUROSCIENCE, BIRKHAEUSER, CAMBRIDGE, MA, US, vol. 19, no. 1/2, August 2002 (2002-08), pages 11-16, XP008031471 ISSN: 0895-8696
- CHESTER A M ET AL: "A Lipophilic Thioflavin-T Derivative for Positron Emission Tomography (PET) Imaging of Amyloid" BIOORGANIC AND MEDICINAL CHEMISTRY LETERS, vol. 12, 2002, pages 295-298, XP002285913
- CHESTER A M: "Synthesis and Evaluation of 11C-Labeled 6-Substituted 2-Arylbenzothiazoles as amyloid Imaging Agents" J. MED. CHEM, vol. 46, 19 June 2003 (2003-06-19), pages 2740-2754, XP002285914

## Description

### FIELD OF THE INVENTION

The present invention relates to thioflavin compounds and specific derivatives that are suitable for imaging amyloid deposits in living patients. More specifically, the present invention relates to a method of imaging amyloid deposits in brain *in vivo* to allow antemortem diagnosis of Alzheimer's disease with thioflavin derivatives. The present invention also relates to therapeutic uses for such compounds.

### BACKGROUND OF THE INVENTION

Alzheimer's Disease ("AD") is a neurodegenerative illness characterized by memory loss and other cognitive deficits. McKhann et al., Neurology 34: 939 (1984). It is the most common cause of dementia in the United States. AD can strike persons as young as 40-50 years of age, yet, because the presence of the disease is difficult to determine without dangerous brain biopsy, the time of onset is unknown. The prevalence of AD increases with age, with estimates of the affected population reaching as high as 40-50% by ages 85-90. Evans et al., JAMA 262: 2551 (1989); Katzman, Necrology 43: 13 (1993).

Studies suggest that amyloid deposition in the brain is an early, causative event in the pathogenesis of Alzheimer's disease (AD). Progression of amyloid deposition results in the formation of neuritic plaques and neurofibrillary tangles in regions of the brain that are involved with learning and memory. A typical Alzheimer's neuritic plaque comprises dystrophic neurites surrounding a core of amyloid material. The principal component of the amyloid core is a protein called amyloid-beta (Aβ).

In practice, AD is definitively diagnosed through examination of brain tissue, usually at autopsy. Khachaturian, Arch. Neurol. 42: 1097 (1985); McKhann et al., Neurology 34: 939 (1984). Neuropathologically, this disease is characterized by the presence of neuritic plaques (NP), neurofibrillary tangles (NFT), and neuronal loss, along with a variety of other findings. Mann, Mech. Ageing Dev. 31: 213 (1985). Post-mortem slices of brain tissue of victims of Alzheimer's disease exhibit the presence of amyloid in the form of proteinaceous extracellular cores of the neuritic plaques that are characteristic of AD.

The amyloid cores of these neuritic plaques are composed of a protein called the β-amyloid (Aβ) that is arranged in a predominately beta-pleated sheet configuration. Mori et al., Journal of Biological Chemistry 267: 17082 (1992); Kirschner et al., PNAS 83: 503 (1986). Neuritic plaques are an early and invariant aspect of the disease. Mann et al., J. Neurol. Sci. 89: 169; Mann, Mech. Ageing Dev. 31: 213 (1985); Terry et al., J. Neuropathol. Exp. Neurol 46: 262 (1987).

The initial deposition of Aβ probably occurs long before clinical symptoms are noticeable. The currently recommended "minimum microscopic criteria" for the diagnosis of AD is based on the number of neuritic plaques found in brain. Khachaturian, *Arch. Neurol., supra* (1985). Assessment of neuritic plaque counts must be delayed until after death, however.

Amyloid-containing neuritic plaques are a prominent feature of selective areas of the brain in AD as well as Down's Syndrome and in persons homozygous for the apolipoprotein E4 allele who are very likely to develop AD. Corder et al., Science 261: 921 (1993); Divry, P., J. Neurol. Psych. 27: 643-657 (1927); Wisniewski et al., in Zimmerman, H.M. (ed.): PROGRESS IN NEUROPATHOLOGY (Grune and Stratton, N.Y. 1973) pp. 1-26.

Brain amyloid is readily demonstrated by staining brain sections with thioflavin S or Congo red. Puchtler et al., J. Histochem. Cytochem. 10: 35 (1962). Congo red-stained amyloid is characterized by a dichroic appearance, exhibiting a yellow-green polarization color. The dichroic binding is the result of the beta-pleated sheet structure of the amyloid proteins. Glenner, G. N. Eng. J. Med. 302: 1283 (1980). A detailed discussion of the biochemistry and histochemistry of amyloid can be found in Glenner, N. Eng. J. Med., 302: 1333 (1980).

Thus far, diagnosis of AD has been achieved mostly through clinical criteria evaluation, brain biopsies and post-mortem tissue studies. Research efforts to develop methods for diagnosing Alzheimer's disease *in vivo* include (1) genetic testing, (2) immunoassay methods and (3) imaging techniques.

Evidence that abnormalities in Aβ metabolism are necessary and sufficient for the development of AD is based on the discovery of point mutations in the Aβ precursor protein in several rare families with an autosomal dominant form of AD. Hardy, Nature Genetics 1: 233 (1992); Hardy et al., Science 256: 184 (1992). These mutations occur near the N- and C-terminal cleavage points necessary for the generation of Aβ from its precursor protein. St. George-Hyslop et al., Science 235: 885 (1987); Kang et al., Nature 325: 733 (1987); Potter WO 92/17152. Genetic analysis of a large number of AD families has demonstrated, however, that AD is genetically heterogeneous. St. George-Hyslop et al., Nature 347: 194 (1990). Linkage to chromosome 21 markers is shown in only some families with early-onset AD and in no families with late-onset AD. More recently a gene on chromosome 14 whose product is predicted to contain multiple transmembrane domains and resembles an integral membrane protein has been identified by Sherrington et al., Nature 375: 754-760 (1995). This gene may account for up to 70% of early-onset autosomal dominant AD. Preliminary data suggests that this chromosome 14 mutation causes an increase in the production of Aβ. Scheuner et al., Soc. Neurosci. Abstr. 21: 1500 (1995). A mutation on a very similar gene has been identified on chromosome 1 in Volga German kindreds with early-onset AD. Levy-Lahad et al., Science 269: 973-977 (1995).

Screening for apolipoprotein E genotype has been suggested as an aid in the diagnosis of AD. Scott, Nature 366: 502 (1993); Roses, Ann. Neurol. 38: 6-14 (1995). Difficulties arise with this technology, however, because the apolipoprotein E4 allele is only a risk factor for AD, not a disease marker. It is absent in many AD patients and present in many nondemented elderly people. Bird, Ann. Neurol. 38: 2-4 (1995).

Immunoassay methods have been developed for detecting the presence of neurochemical markers in AD patients and to detect an AD related amyloid protein in cerebral spinal fluid. Warner, Anal. Chem. 59: 1203A (1987); World Patent No. 92/17152 by Potter; Glenner et al., U.S. Patent No. 4,666,829. These methods for diagnosing AD have not been proven to detect AD in all patients, particularly at early stages of the disease and are relatively invasive, requiring a spinal tap. Also, attempts have been made to develop monoclonal antibodies as probes for imaging of Aβ. Majocha et al., J. Nucl. Med., 33: 2184 (1992); Majocha et al., WO 89/06242 and Majocha et al., U.S. Patent 5,231,000. The major disadvantage of antibody probes is the difficulty in getting these large molecules across the blood-brain barrier. Using antibodies for *in vivo* diagnosis of AD would require marked abnormalities in the blood-brain barrier in order to gain access into the brain. There is no convincing functional evidence that abnormalities in the blood-brain barrier reliably exist in AD. Kalaria, Cerebrovascular & Brain Metabolisin Reviews 4: 226 (1992).

Radiolabeled Aβ peptide has been used to label diffuse, compact and neuritic type plaques in sections of AD brain. See Maggio et al., WO 93/04194. However, these peptides share all of the disadvantages of antibodies. Specifically, peptides do not normally cross the blood-brain barrier in amounts necessary for imaging and because these probes react with diffuse plaques, they may not be specific for AD.

Neuritic plaques and neurofibrillary tangles are the two most characteristic pathological hallmarks of AD. Klunk and Abraham, Psychiatric Development, 6:121-152 (1988). Plaques occur earliest in neocortex where they are relatively evenly distributed. Thal et al., Neurology 58:1791-1800 (2002). Tangles appear first in limbic areas such as the transentorhinal cortex and progress in a predictable topographic pattern to the neocortex. Braak and Braak, Acta Neuropathologica 82:239-259 (1991). Arnold *et al.* mapped the distribution of NFT and neuritic plaques in the brains of patients with AD. Arnold et al., Cereb. Cortex 1:103-116 (1991). Compared to NFT, neuritic plaques were, in general, more evenly distributed throughout the cortex, with the exceptions of notably fewer neuritic plaques in limbic periallocortex and allocortex (the areas with greatest NFT density). By thioflavin-S staining, temporal and occipital lobes had the highest neuritic plaque densities, limbic and frontal lobes had the lowest, and parietal lobe was intermediate. Arriagada et al., Neurology 42:1681-1688 (1992). Arriagada *et al* studied the topographic distribution of AD-type pathologic changes in the brains of presumed nondemented elderly individuals. Their observations suggest that most individuals over the age of 55 have at least a few NFT and plaques. Immunohistochemically defined subtypes of SP had distinct patterns of distribution with Aβ-immunoreactive plaques present in neocortical areas much greater than limbic areas and Alz-50 immunoreactive plaques being infrequent and limited to those areas that contain Alz-50-positive neurons and NFT. These patterns suggested a commonality in the pathologic processes that lead to NFT and SP in both aging and AD.

There remains debate as to whether plaques and tangles are byproducts of the neurodegenerative process found in AD or whether they are the cause of neuronal cell death. Ross, Current Opinion in Neurobiol. 96:644-650 (1996); Terry, J. of Neuropath. & Exp. Neurol. 55:1023-1025 (1996); Terry, J Neural Transmission- Suppl. 53:141-145 (1998). Evidence is clear that neocortical and hippocampal synapse loss correlates well with premorbid cognitive status. Some researchers suggest that disruption of microtubule structure and function, caused by the hyperphosphorylation of the microtubule-associated protein, tau, plays the key etiologic role in synapse loss in particular and AD in general. Terry, J. of Neuropath. & Exp. Neurol. 55:1023-1025 (1996); Terry, J of Neural Transmission - Suppl. 53:141-145 (1998). Oxidative damage and membrane breakdown have been proposed to play important roles in AD. Perry, Free Radical Biology & Medicine 28:831-834 (2000); Pettegrew et al., Annals of the New York Academy of Sciences 826:282-306 (1997). Vascular factors including subtle, chronic cerebral hypoperfusion also have been implicated in the pathogenesis of AD. De la Torre, Annals of the New YorkAcademy of Sciences 903:424-436 (2000); Di Iorio et al., Aging (Milano) 11:345-352 (1999). While all of these factors are likely to play some role in the pathogenesis of AD, increasing evidence points to abnormalities in the processing of the amyloid-beta (Aβ) peptide, a 4 kD peptide that aggregates into a fibrillar, β-pleated sheet structure. Glenner and Wong, Biochemical & Biophysical Research Communications 120:885-890 (1984). Aβ has been proposed to play an important role in the pathogenesis of AD for several reasons: 1) Aβ deposits are the earliest neuropathological markers of AD in Down's Syndrome, and can precede NFT formation by several decades Mann et al., Neurodegeneration 1:201-215 (1992); Naslund, et al., JAMA 283:1571-1577 (2000). 2) β-amyloidosis is relatively specific to AD and closely related disorders; Selkoe, Trends in Neurosciences 16:403-409 (1993); 3) Aβ is toxic to cultured neurons, Yankner Neurobiol.Aging 13:615-616 (1992); Mattson et al., J. Neuroscience 12:376-389 (1992); Shearman et al., Proc.Natl.Acad.Sci. USA 91:1470-1474 (1994), a toxicity that appears to be dependent on β-sheet secondary structure and aggregation into at least oligomers. Lambert et al. Proc.Natl.Acad.Sci. USA 95:6448-6453 (1989) ; Pike et al., J. Neuroscience 13:1676-1687 (1993) ; Simmons et al., Molecular Pharmacology 45:373-379 (1994). Although Aβ surely exists in an equilibrium distributed across monomeric, oligomeric and fibrillar/plaque fractions, the oligomeric form of Aβ has been strongly implicated as the key neurotoxic component. Selkoe, Alzheimer disease, edited by R.D.Terry, et al, pp. 293-310 Lippincott Williams and Wilkins, Philadelphia (1999); Selkoe, Science 298, 789-91 (2002). Recognition of the toxic effects of oligomeric Aβ has formed a basis for compromise for some opponents of the "amyloid cascade hypothesis" of AD. Terry, Ann. Neurol. 49:684 (2001). Perhaps the strongest evidence for a role of Aβ in the pathogenesis of AD comes from the finding of mutations in the amyloid precursor protein (APP) gene which lead to some forms of early onset familial AD. Goate et al., Nature 349:704-706 (1991). In addition, all familial forms of autosomal dominant AD have in common an elevated level of the more rapidly aggregating 42 amino acid form of Aβ. Younkin Rinsho Shinkeigaku - Clinical Neurology 37:1099 (1997). In contrast, no mutation in the tau protein has been shown to cause AD. Instead mutations in tau (chromosome 17) are linked to frontotemporal dementia with Parkinsonism. Goedert et al., Neuron 21:955-958 (1998). Recent evidence has shown a good correlation between the levels of Aβ in brain and cognitive decline in AD and the deposition of amyloid appears to be a very early, perhaps the first, event in the pathogenesis of AD, preceding any cognitive impairment. Naslund, et al., JAMA 283:1571-1577 (2000). Its presence may modulate a number of biochemical pathways that result in the deposition of still other proteins, the activation of astroglia and microglia, and eventually neuronal cell death and consequent cognitive dysfunction.

Data suggest that amyloid-binding compounds will have therapeutic potential in AD and type 2 diabetes mellitus. Morphological reactions including, reactive astrocytosis, dystrophic neurites, activated microglia cells, synapse loss, and full complement activation found around neuritic plaques all signify that neurotoxic and cell degenerative processes are occurring in the areas adjacent to these Aβ deposits. Joachim et al., Am. J. Pathol. 135: 309 (1989); Masliah *et al., loc. cit.* 137: 1293 (1990); Lue and Rogers, Dementia 3: 308 (1992). Aβ-induced neurotoxicity and cell degeneration has been reported in a number of cell types *in vitro.* Yankner et al., Science 250: 279 (1990); Roher et al., BBRC 174: 572 (1991); Frautschy et al., Proc. Natl. Acad. Sci. 88: 83362 (1991); Shearman *et al., loc. cit.* 91: 1470 (1994). It has been shown that aggregation of the Aβ peptide is necessary for *in vitro* neurotoxicity. Yankner, Neurobiol. Aging 13: 615 (1992). Recently, three laboratories have reported results which suggest that Congo red inhibits Aβ-induced neurotoxicity and cell degeneration *in vitro.* Burgevin et al., NeuroReport 5: 2429 (1994); Lorenzo and Yankner, Proc. Natl. Acad. Sci. 91: 12243 (1994); Pollack et al., Neuroscience Letters 184: 113 (1995); Pollack et al., Neuroscience Letters 197: 211 (1995). The mechanism appears to involve both inhibition of fibril formation and prevention of the neurotoxic properties of formed fibrils. Lorenzo and Yankner, Proc. Natl. Acad. Sci. 91: 12243 (1994). Congo red also has been shown to protect pancreatic islet cells from the toxicity caused by amylin. Lorenzo and Yankner, Proc. Natl. Acad. Sci. 91: 12243 (1994). Amylin is a fibrillar peptide similar to Aβ which accumulates in the pancreas in type 2 diabetes mellitus.

It is known in the art that certain azo dyes, such as Congo red, may be carcinogenic. Morgan et al. Environmental Health Perspectives, 102 (supp.) 2: 63-78, (1994). This potential carcinogenicity appears to be based largely on the fact that azo dyes are extensively metabolized to the free parent amine by intestinal bacteria. Cerniglia et al., Biochem. Biophys. Res. Com., 107: 1224-1229, (1982). In the case of benzidine dyes (and many other substituted benzidines), it is the free amine which is the carcinogen. These facts have little implications for amyloid imaging studies in which an extremely minute amount of the high specific activity radiolabelled dye would be directly injected into the blood stream. In this case, the amount administered would be negligible and the dye would by-pass the intestinal bacteria.

In the case of therapeutic usage, these facts have critical importance. Release of a known carcinogen from a therapeutic compound is unacceptable. A second problem with diazo dye metabolism is that much of the administered drug is metabolized by intestinal bacteria prior to absorption. This lowered bioavailability remains a disadvantage even if the metabolites released are innocuous.

Thioflavin T is a basic dye first described as a selective amyloid dye in 1959 by Vassar and Culling, Arch. Pathol. 68: 487 (1959). Schwartz et al., Zbl. Path. 106: 320 (1964), first demonstrated the use of Thioflavin S, an acidic dye, as an amyloid dye in 1964. The properties of both Thioflavin T and Thioflavin S have since been studied in detail. Kelenyi, J. Histochem. Cytochem. 15: 172 (1967); Burns et al., J. Path. Bact. 94:337 (1967); Guntern et al., Experientia 48: 8 (1992); LeVine, Meth. Enzymol. 309: 274 (1999). Thioflavin S commonly is used in the post-mortem study of amyloid deposition in AD brain where it has been shown to be one of the most sensitive techniques for demonstrating senile plaques. Vallet et al., Acta Neuropathol. 83: 170 (1992). Thioflavin T has been frequently used as a reagent to study the aggregation of soluble amyloid proteins into beta-sheet fibrils. LeVine, Prot. Sci. 2: 404 (1993). Quaternary amine derivatives related to Thioflavin T have been proposed as amyloid imaging agents, although no evidence of brain uptake of these agents has been presented. Caprathe et al., U.S. Patent No. 6,001,331.

Since the initial deposition of amyloid may occur long before clinical symptoms of AD are noticeable, the detection and quantitation of amyloid deposits could facilitate the diagnosis of AD in its early, pre-symptomatic stages. *See* U.S. Patent No. 6,417,178. Imaging techniques, such as positron emission tomography (PET) and single photon emission computed tomography (SPECT), are effective in monitoring the accumulation of amyloid deposits in the brain and correlating it to the progression of AD. The application of these techniques requires the development of radioligands that readily enter the brain and selectively bind to amyloid deposits *in vivo.*

Wang et al, J. of Mol. Neurosci. 19, 11 (2002), discloses the use of 2-(3'-Iodo-4'aminophenyl)-6-hydroxybenzothiazole radiolabelled with ¹²⁵I for in vivo quantitation of amyloid deposits.

The inability to assess amyloid deposition in AD until after death impedes the study of this devastating illness. A method of quantifying amyloid deposition before death is needed both as a diagnostic tool in mild or clinically confusing cases as well as in monitoring the effectiveness of therapies targeted at preventing Aβ deposition. Therefore, it remains of utmost importance to develop a safe and specific method for diagnosing AD before death by imaging amyloid in brain parenchyma *in vivo.* Even though various attempts have been made to diagnose AD in vivo, currently, there are no antemortem probes for brain amyloid. No method has utilized a high affinity probe for amyloid that has low toxicity, can cross the blood-brain barrier, and binds more effectively to AD brain than to normal brain in order to identify AD amyloid deposits in brain before a patents death. Thus, no *in vivo* method for AD diagnosis has been demonstrated to meet these criteria.

A need exists for amyloid binding compounds that are non-toxic and can cross the blood-brain barrier, and, consequently, can be used in diagnostics. To that end, the present inventors have determined that varying substitution in different positions can increase binding affinity depending upon position of the substituent.

In addition, the present inventors have developed a series of uncharged derivatives of thioflavin T as amyloid-imaging agents that exhibit high affinity for amyloid deposits and high permeability across the blood-brain barrier. Extensive in vitro and in vivo studies of these amyloid-imaging agents suggest that they specifically bind to amyloid deposits at concentrations typical of those achieved during positron emission tomography studies. In the complex milieu of human brain, non-specific binding of the amyloid-imaging compounds is low, even in control brains devoid of amyloid deposits. At nanomolar concentration, these compounds appear not to bind to neurofibrillary tangles.

### SUMMARY OF THE INVENTION

It is therefore an embodiment of the present invention to provide compounds which allow for a safe and specific method for diagnosing AD before death by in vivo imaging of amyloid in brain parenchyma. The compounds useful for this purpose are according to the following formulax I: 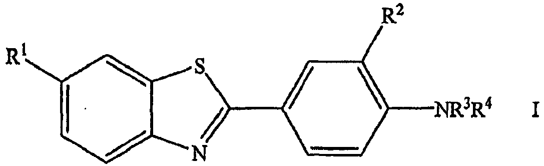 or a pharmaceutically acceptable salt, hydrate, solvate or prodrug of the compound, wherein:
R¹ is hydrogen, -OH, -NO₂, -CN, -COOR, -OCH₂OR, C₁-C₆ alkyl, C₂-C₆ alkenyl, C₂-C₆ alkynyl, C₁-C₆ alkoxy or halo, wherein one or more of the atoms of R¹ may be a radiolabeled atom;
R is C₁-C₆ alkyl, wherein one or more of the carbon atoms may be a radiolabeled atom;
R² is a radiofluoro;
R³ is hydrogen, C₁-C₆ alkyl, C₂-C₆ alkenyl or C₂-C₆ alkynyl; and
R⁴ is hydrogen, C₁-C₆ alkyl, C₂-C₆ alkenyl or C₂-C₆alkynyl.

In another embodiment, the amyloid binding compounds of the invention bind to Aβ with a dissociation constant (K_{D}) between 0.0001 and 10.0µM when measured by binding to synthetic Aβ peptide or Alzheimer's Disease brain tissue.

A method for synthesizing the amyloid binding compounds of the present invention having at least one of the substituents selected from the group consisting of ¹⁸F, and ¹⁹F comprises the step of labeling the amyloid binding compound wherein at least one of the substituents is a tri-alkyl tin, by reaction of the compound with a ¹⁸F, or ¹⁹F containing substance.

A further embodiment of the present invention relates to a pharmaceutical composition for *in vivo* imaging of amyloid deposits, comprising (a) an amyloid binding compound of the present invention and (b) a pharmaceutically acceptable carrier.

The invention enables an *in vivo* method for detecting amyloid deposits in a subject, comprising the steps of: (a) administering a detectable quantity of a pharmaceutical composition comprising the labeled amyloid binding compound, and detecting the binding of the compound to amyloid deposit in the subject. In a preferred aspect of this embodiment, the amyloid deposit is located in the brain of a subject. In a particularly preferred aspect of this embodiment, the subject is suspected of having a disease or syndrome selected from the group consisting of Alzheimer's Disease, familial Alzheimer's Disease, Down's Syndrome and homozygotes for the apolipoprotein E4 allele. In another particularly preferred aspect of this embodiment, the detecting is selected from the group consisting of gamma imaging, magnetic resonance imaging and magnetic resonance spectroscopy. In a preferred embodiment of the method the gamma imaging is either PET or SPECT. In another preferred embodiment of the method, the pharmaceutical composition is administered by intravenous injection. In another preferred embodiment of the method, the ratio of (i) binding of the compound to a brain area other than the cerebellum to (ii) binding of the compound to the cerebellum, in a subject is compared to the ratio in a normal subject.

In another embodiment of the method, the detecting of amyloid deposits *in vivo* is used in the diagnosis of Alzheimer's disease.

The invention also enables a method of detecting amyloid deposits in biopsy or post-mortem human or animal tissue comprising the steps of: (a) incubating formalin-fixed or fresh-frozen tissue with a solution of an amyloid binding compound of the present invention to form a labeled deposit and then, (b) detecting the labeled deposits. In a preferred aspect of this embodiment, the solution is composed of 25-100% ethanol, with the remainder of the solution being water, wherein the solution is saturated with an amyloid binding compound according to the present invention. In a particularly preferred embodiment of this method, the solution is composed of an aqueous buffer (such as tris or phosphate) containing 0-50% ethanol, wherein the solution contains 0.0001 to 100 µM of an amyloid binding compound according to the present invention. In a particularly preferred embodiment of the method, the detecting is effected by microscopic techniques selected from the group consisting of bright-field, fluorescence, laser-confocal, and cross-polarization microscopy.

The invention further enables a method of quantifying the amount of amyloid in biopsy or post-mortem tissue comprising the steps of: a) incubating a radiolabeled derivative of an amyloid binding compound of the present invention with a homogenate of biopsy or post-mortem tissue, b) separating the tissue-bound from the tissue-unbound radiolabeled derivative of an amyloid binding compound of the present invention, c) quantifying the tissue-bound radiolabeled derivative of an amyloid binding compound of the present invention, and d) converting the units of tissue-bound radiolabeled derivative of an amyloid binding compound of the present invention to units of micrograms of amyloid per 100 mg of tissue by comparison with a standard.

The invention also enables a method of distinguishing an Alzheimer's disease brain from a normal brain comprising the steps of: a) obtaining tissue from (i) the cerebellum and (ii) another area of the same brain other than the cerebellum, from normal subjects and from subjects suspected of having Alzheimer's disease; b) incubating the tissues with a radiolabeled derivative of a thioflavin amyloid binding compounds according to the present invention so that amyloid in the tissue binds with the radiolabeled derivative of an amyloid binding compound of the present invention; c) quantifying the amount of amyloid bound to the radiolabeled derivative of an amyloid binding compound of the present invention according to the above recited method; d) calculating the ratio of the amount of amyloid in the area of the brain other than the cerebellum to the amount of amyloid in the cerebellum; e) comparing the ratio for amount of amyloid in the tissue from normal subjects with ratio for amount of amyloid in tissue from subjects suspected of having Alzheimer's disease; and f) determining the presence of Alzheimer's disease if the ratio from the brain of a subject suspected of having Alzheimer's disease is above 90% of the ratios obtained from the brains of normal subjects.

Another embodiment of the present invention relates to compounds of the present invention which are useful in binding specifically to amyloid deposits over neurofibrillary tangles.

The invention also enables a method of selectively binding to amyloid plaques but not to neurofibrillary tangles *in vivo* brain tissue which contains both by administering an effective amount of a compound of the present invention so that blood concentration of the administered compound remains below 10 nM *in vivo*.

Another embodiment relates to the use of a compound of Formula I for the preparation of a medicament for diagnosis of Alzheimer's disease in a patient in need thereof.

Other embodiments of the invention will be apparent to those skilled in the art from consideration of the specification and practice of the invention disclosed herein. It is intended that the specification be considered as exemplary only, with the true scope and spirit of the invention being indicated by the following claims. Additionally, all documents referred to herein are expressly incorporated by reference.

### BRIEF DESCRIPTION OF THE DRAWINGS

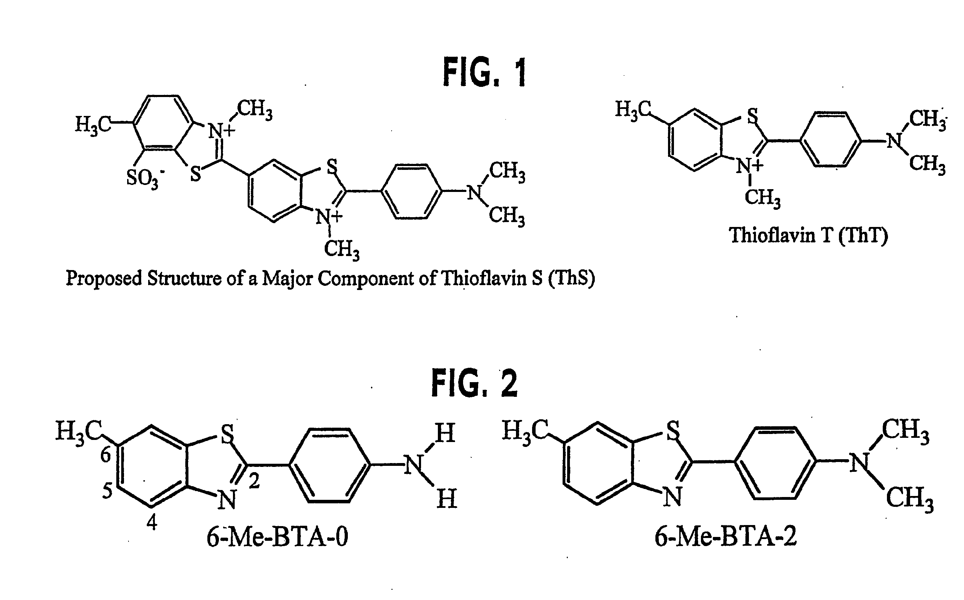

Figure 1 Shows the structures of a Thioflavin S and Thioflavin T;
Figure 2 Shows the structures of two Thioflavin derivatives according to the invention;

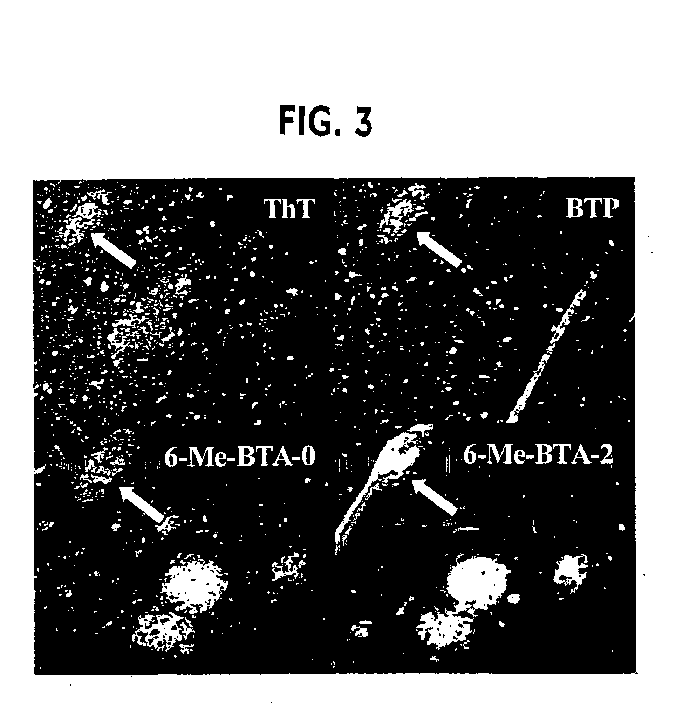

Figure 3 Shows four serial sections of fluorescent dyed brain frontal cortex of an AD patient;

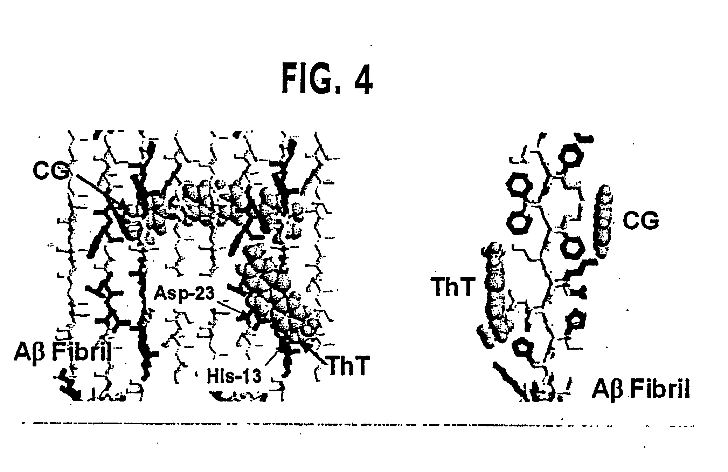

Figure 4 Shows proposed sites of binding of Chrysamine G and Thioflavin T in β-sheet fibrils;
Figure 5 Shows competition assay using Chrysamine G, Thioflavin S and Thioflavin T, and derivatives of the present invention (BTA-0, BTA-1 and BTA-2);

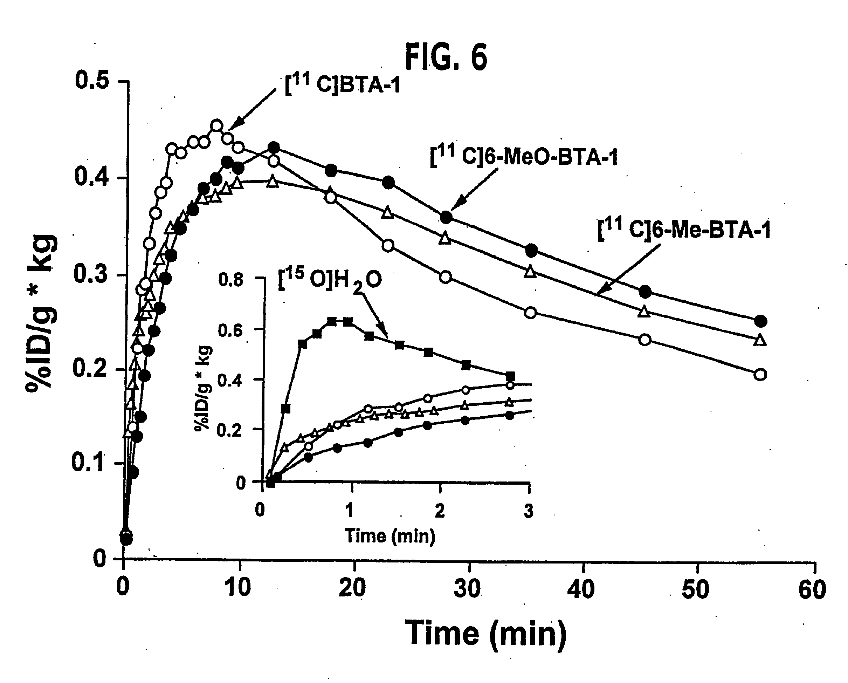

Figure 6 Shows time course radioactivity in the frontal cortex of baboons injected with labeled BTA-1, 6-MeO-BTA-1 and 6-Me-BTA-1; and

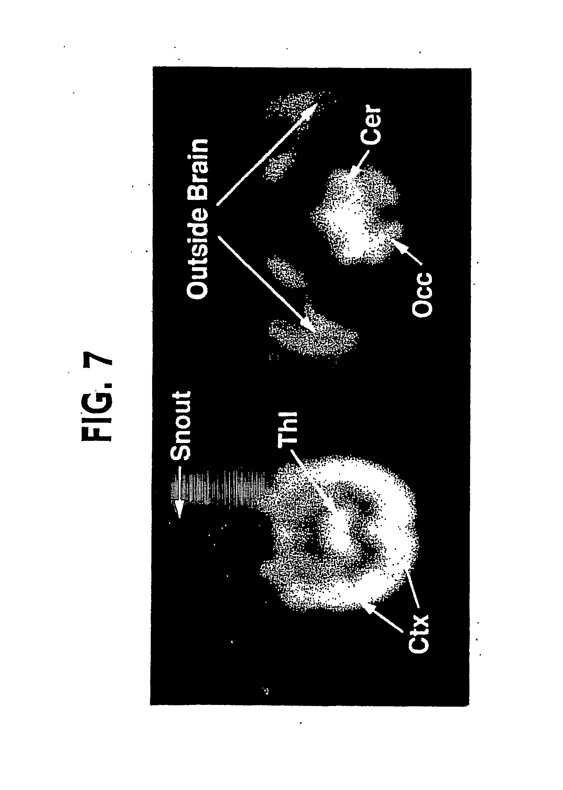

Figure 7 Shows a tranverse positron emission tomography image of two levels of baboon brain following i.v. injection of [N-methyl-¹¹C]BTA-1.
Figure 8 Shows post-mortem sections of human and transgenic mouse brain stained with a derivative of the present invention (BTA-1).
Figure 9 Shows in vivo labeling of amyloid plaques and vascular amyloid stained by a derivative of the present invention (BTA-1) in living transgenic mice imaged with multiphoton microscopy.

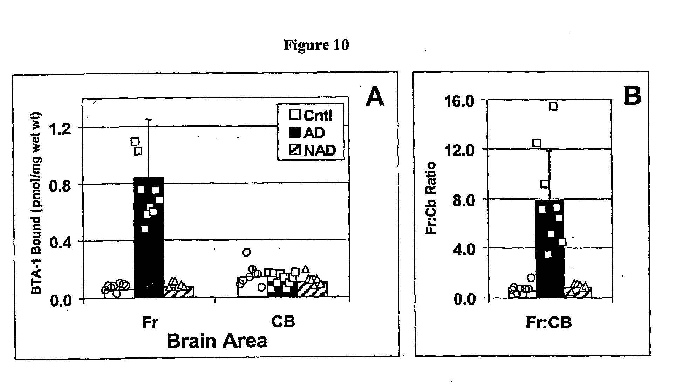

Figure 10A Shows a data comparison of [³H]binding to homogenates from a control brain, Alzheimer's disease brain and non-Alzheimer's disease dementia brain.
Figure 10B Shows data ratio from a comparison of the frontal cortex and cerebellum for each individual brain in a control brain, Alzheimer's disease brain and non-Alzheimer's disease dementia brain.
Figure 11A-F Shows the specificity of the inventive compounds for amyloid plaques over neurofibrillary tangles, where A and B show the entorhinal cortex, C and D show the frontal cortex and E and F show the cerebellum of a Braak stage II control crain.

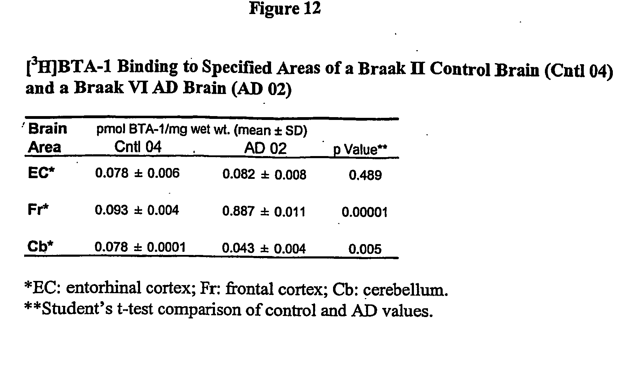

Figure 12 Table showing [³H] BTA-1 binding to specified areas of a Braak II Control Brain and a Braak VI AD Brain (AD02).

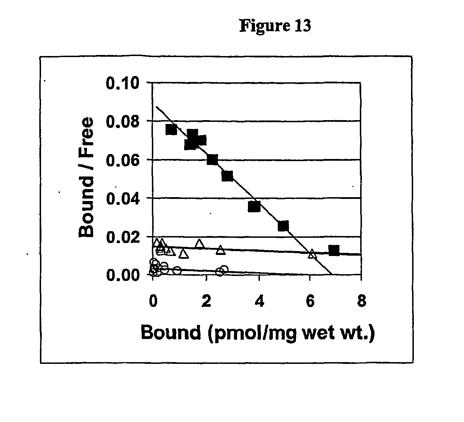

Figure 13 Shows a Scratchard plot of the binding of [³H] BTA-1 to homogenates from AD frontal gray matter and underlying frontal white matter from the same AD brain.

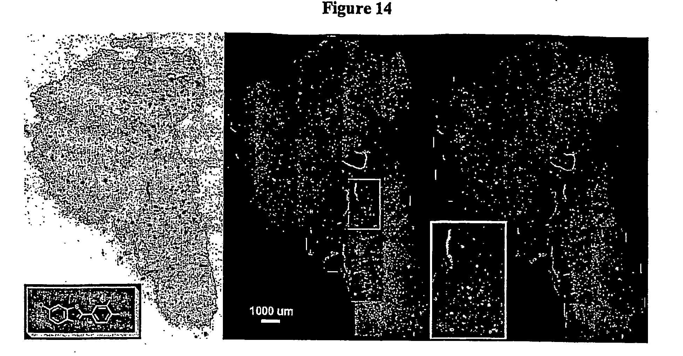

Figure 14 Shows an autoradiogram and fluorescent micrograph showing the overlap of [I-125]6-OH-BTA-0-3'-I binding to plaques and cerebrovascular amyloid and amyloid deposits stained by the amyloid dye, X-34. Left: autoradiogram of [I-125]6-OH-BTA-0-3'-I binding to fresh frozen tissue from post-mortem AD brain. The dark areas show the localization of [I-125]6-OH-BTA-0-3'-I and are outlined in red. The structure of [I-125]6-OH-BTA-0-3'-I is shown in the lower left. Right: The same piece of post-mortem AD brain tissue stained with the amyloid dye, X-34. Bright areas indicate plaques and cerebrovascular amyloid. Center: Overlap of the red outline from the left autoradiogram with the X-34 stain showing nearly 1:1 correspondence of [I-125]6-OH-BTA-0-3'-I binding to plaques and cerebrovascular amyloid. Inset: Shows a 2.25-fold enlargement of the boxed area in the center figure. Bar represents 1000 µm.
Figure 15 Shows time-activity curves of the penetration and clearance of radioactivity from three regions of baboon brain following the injection of Compound B (2-(3-[¹⁸F]-fluoro-4-methylamino-phenyl)-benzothiazol-6-ol).
Figure 16 Shows time-activity curves of the penetration and clearance of radioactivity from baboon cerebellum (reference region devoid of specific binding) following the injection of the radioligands [carbonyl-¹¹C]WAY100635, [¹¹C](+)-McN5652, and [¹⁸F]altanserin compared to the behavior of Compound B.
Figure 17 Shows time-activity curves of the penetration and clearance of radioactivity from three regions of baboon brain following the injection of Compound C (2-[4-(3-¹⁸F-fluoro-propylamino)-phenyl]benzothiazol-6-ol).
Figure 18 Shows time-activity curves of the penetration and clearance of radioactivity from baboon cerebellum (reference region devoid of specific binding) following the injection of the radioligands [carbonyl-¹¹C]WAY100635, [¹¹C](+)-McN5652, and [¹⁸F]altanserin compared to the behavior of Compound C.

### DETAILED DESCRIPTION

The present invention exploits the ability of Thioflavin compounds and radiolabeled derivatives thereof to cross the blood brain barrier *in vivo* and bind to Aβ deposited in neuritic (but not diffuse) plaques, to Aβ deposited in cerebrovascular amyloid, and to the amyloid consisting of the protein deposited in NFT. The present compounds are nonquaternary amine derivatives of Thioflavin S and T which are known to stain amyloid in tissue sections and bind to synthetic Aβ in vitro. Kelenyi, J. Histochem. Cytochem. 15: 172 (1967); Burns et al., J. Path. Bact. 94:337 (1967); Guntern et al., Experientia 48: 8 (1992); LeVine, Meth. Enzymol. 309: 274 (1999).

The thioflavin derivatives of the present invention have each of the following characteristics: (1) specific binding to synthetic Aβ *in vitro* and (2) ability to cross a noncompromised blood brain barrier *in vivo.*

### Molecular Modeling

Molecular modeling was done, using the computer modeling program Alchemy2000, a product of Tripost, Inc. (St. Louis, MO), to generate the Aβ peptide chains in the antiparallel beta-sheet conformation. Kirschner et al., Proc. Natl. Acad. Sci. U.S.A. 83: 503 (1986). The amyloid peptides were placed in hairpin loops, see Hilbich et al., J. Mol. Biol. 218: 149 (1991), and were used without further structural refinement. The Aβ peptides were aligned so that alternate chains were spaced 4.76 A apart, characteristic of beta-sheet fibrils. Kirschner, *supra.* Thioflavin T derivatives were energy minimized and aligned with the fibril model to maximize contact with Asp-23/Gln-15/His-13 of Aβ(1-42)

### Characterization of Specific Binding to Aβ Synthetic Peptide: Affinity, Kinetics, Maximum Binding

The characteristics of thioflavin derivative binding were analyzed using synthetic Aβ(1-40) and 2-(4'-[¹¹C]methylamino-phenyl)-benzothiazole ([N-methyl-¹¹C]BTA-1) in phosphate-buffered saline (pH 7.0) or glycine buffer/20% ethanol (pH 8.0), as previously described for Chysamine-G binding. Klunk et al., Neurobiol. Aging 15: 691 (1994).

### Amino acid sequence for Aβ(1-40) is as follows:

| **1** | **2** | **3** | **4** | **5** | **6** | **7** | **8** | **9** | **10** | **11** | **12** |
|---|---|---|---|---|---|---|---|---|---|---|---|
| Asp | Ala | Glu | Phe | Arg | His | Asp | Ser | Gly | Tyr | Glu | Val |
| | | | | | | | | | | | |

| **13** | **14** | **15** | **16** | **17** | **18** | **19** | **20** | **21** | **22** | **23** | **24** |
|---|---|---|---|---|---|---|---|---|---|---|---|
| His | His | Gln | Lys | Leu | Val | Phe | Phe | Ala | Glu | Asp | Val |
| | | | | | | | | | | | |

| **25** | **26** | **27** | **28** | **29** | **30** | **31** | **32** | **33** | **34** | **35** | **36** |
|---|---|---|---|---|---|---|---|---|---|---|---|
| Gly | Ser | Asn | Lys | Gly | Ala | Ile | Ile | Gly | Leu | Met | Val |
| | | | | | | | | | | | |

| **37** | **38** | **39** | **40** | | | | | | | | |
|---|---|---|---|---|---|---|---|---|---|---|---|
| Gly | Gly | Val | Val | | | | | | | | |

"Alkyl" refers to a saturated straight or branched chain hydrocarbon radical. Examples include without limitation methyl, ethyl, propyl, iso-propyl, butyl, iso-butyl, *tert-*butyl, n-pentyl and n-hexyl.

"Alkenyl" refers to an unsaturated straight or branched chain hydrocarbon radical comprising at least one carbon to carbon double bond. Examples include without limitation ethenyl, propenyl, iso-propenyl, butenyl, iso-butenyl, *tert*-butenyl, n-pentenyl and n-hexenyl.

"Alkynyl" refers to an unsaturated straight or branched chain hydrocarbon radical comprising at least one carbon to carbon triple bond. Examples include without limitation ethynyl, propynyl, iso-propynyl, butynyl, iso-butynyl, *tert*-butynyl, pentynyl and hexynyl.

"Alkoxy" refers to an alkyl group bonded through an oxygen linkage. "Halo" refers to a fluoro, chloro, bromo or iodo radical.

"Effective amount" refers to the amount required to produce a desired effect. Examples of an "effective amount" include amounts that enable imaging of amyloid deposit(s) *in vivo*, that yield acceptable toxicity and bioavailability levels for pharmaceutical use, and/or prevent cell degeneration and toxicity associated with fibril formation.

"Pharmaceutically acceptable carrier" refers to a pharmaceutically acceptable material, composition or vehicle, such as a liquid or solid filler, diluent, excipient or solvent encapsulating material, involved in carrying or transporting the subject compound from one organ, or portion of the body, to another organ or portion of the body. Each carrier is "acceptable" in the sense of being compatible with the other ingredients of the formulation and suitable for use with the patient. Examples of materials that can serve as a pharmaceutically acceptable carrier include without limitation: (1) sugars, such as lactose, glucose and sucrose; (2) starches, such as corn starch and potato starch; (3) cellulose and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) pH buffered solutions; (21) polyesters, polycarbonates and/or polyanhydrides; and (22) other non-toxic compatible substances employed in pharmaceutical formulations as identified, for example, in REMINGTON's PHARMACEUTICAL SCIENCES, 15th ed. (Mack Publishing Co., 1975), at pages 1405-1412 and 1461-1487, and THE NATIONAL FORMULARY XIV, 14th ed. (American Pharmaceutical Association, 1975).

"Pharmaceutically acceptable salt" refers to an acid or base salt of the inventive compound, which salt possesses the desired pharmacological activity and is neither biologically nor otherwise undesirable. The salt can be formed with acids that include without limitation acetate, adipate, alginate, aspartate, benzoate, benzenesulfonate, bisulfate butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride hydrobromide, hydroiodide, 2-hydroxyethanesulfonate, lactate, maleate, methanesulfonate, 2-naphthalenesulfonate, nicotinate, oxalate, thiocyanate, tosylate and undecanoate. Examples of a base salt include without limitation ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such as arginine and lysine. In some embodiments, the basic nitrogen-containing groups can be quartemized with agents including lower alkyl halides such as methyl, ethyl, propyl and butyl chlorides, bromides and iodides; dialkyl sulfates such as dimethyl, diethyl, dibutyl and diamyl sulfates; long chain halides such as decyl, lauryl, myristyl and stearyl chlorides, bromides and iodides; and aralkyl halides such as phenethyl bromides.

"Prodrug" refers to a derivative of the inventive compound that undergoes biotransformation, such as metabolism, before exhibiting its pharmacological effect(s). The prodrug is formulated with the objective(s) of improved chemical stability, improved patient acceptance and compliance, improved bioavailability, prolonged duration of action, improved organ selectivity, improved formulation (e.g., increased hydrosolubility), and/or decreased side effects (e.g., toxicity). The prodrug can be readily prepared from the inventive compound using conventional methods, such as that described in BURGER'S MEDICINAL CHEMISTRY AND DRUG CHEMISTRY, 5th ed., Vol. 1 (1995), pages 172-178 and 949-982.

"Animal" refers to a living organism having sensation and the power of voluntary movement, and which requires for its existence oxygen and organic food. Examples include, without limitation, members of the human, equine, porcine, bovine, murine, canine and feline species. In the case of a human, an "animal" also may be referred to as a "patient."

"Mammal" refers to a warm-blooded vertebrate animal.

"Treating" refers to:
(i) preventing a disease, disorder or condition from occurring in an animal that may be predisposed to the disease, disorder and/or condition but has not yet been diagnosed as having it;
(ii) inhibiting the disease, disorder or condition, i.e., arresting its development; and/or
(iii) relieving the disease, disorder or condition, i.e., causing regression of the disease, disorder and/or condition.

Unless the context clearly dictates otherwise, the definitions of singular terms may be extrapolated to apply to their plural counterparts as they appear in the application; likewise, the definitions of plural terms may be extrapolated to apply to their singular counterparts as they appear in the application.

### COMPOUNDS

This invention provides a radiolabeled compound of formula I 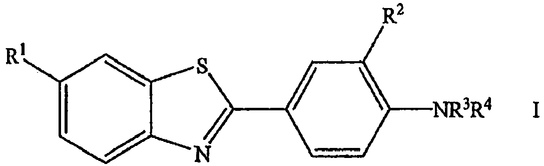 or a pharmaceutically acceptable salt, hydrate, solvate or prodrug of the compound, wherein:
R¹ is hydrogen, -OH, -NO₂, -CN, -COOR, -OCH₂OR, C₁-C₆ alkyl, C₂-C₆ alkenyl, C₂-; C₆ alkynyl, C₁-C₆ alkoxy or halo, wherein one or more of the atoms of R¹ may be a radiolabeled atom;
R is C₁-C₆ alkyl, wherein one or more of the carbon atoms may be a radiolabeled atom;
R² is a radiofluoro;
R³ is hydrogen, C₁-C₆ alkyl, C₂-C₆ alkenyl or C₂-C₆ alkynyl; and
R⁴ is hydrogen, C₁-C₆ alkyl, C₂-C₆ alkenyl or C₂-C₆ alkynyl,

Examples of a radiolabeled carbon include, without limitation, ¹¹C, ¹³C and ¹⁴C. In one embodiment, R¹ is -OH.

As indicated, R² is a radiofluoro. As an example of the compounds, R¹ is -OH or C₁-C₆ alkoxy; R² is ¹⁸F; and R³ and R⁴ are independently hydrogen or C₁-C₆ alkyl. As another example, R¹ is -OH; R³ is hydrogen; and R⁴ is -CH₃.

In yet another embodiment, the inventive compounds bind selectively to amyloid, particularly synthetic Aβ *in vitro* or Aβ deposited in neuritic plaques; cross a noncompromised blood-brain barrier *in vivo*; are bioavailable; and/or are non-toxic.

### METHODS OF USE

The inventive compounds may be used to determine the presence, location and/or amount of one or more amyloid deposit(s) in an organ or body area, including the brain, of an animal. Amyloid deposit(s) include, without limitation, deposit(s) of Aβ. In allowing the temporal sequence of amyloid deposition to be followed, the inventive compound may further be used to correlate amyloid deposition with the onset of clinical symptoms associated with a disease, disorder or condition. The inventive compounds may ultimately be used to, and to diagnose a disease, disorder or condition characterized by amyloid deposition, such as AD, familial AD, Down's syndrome, amyloidosis, Type II diabetes mellitus, and homozygotes for the apolipoprotein E4 allele.

The method of this invention determines the presence and location of amyloid deposits in an organ or body area, preferably brain, of a patient. The present method comprises administration of a detectable quantity of a pharmaceutical composition containing an amyloid binding compound of the present invention called a "detectable compound," or a pharmaceutically acceptable water-soluble salt thereof, to a patient. A "detectable quantity" means that the amount of the detectable compound that is administered is sufficient to enable detection of binding of the compound to amyloid. An "imaging effective quantity" means that the amount of the detectable compound that is administered is sufficient to enable imaging of binding of the compound to amyloid.

The invention employs amyloid probes which, in conjunction with non-invasive neuroimaging techniques such as magnetic resonance spectroscopy (MRS) or imaging (MRI), or gamma imaging such as positron emission tomography (PET) or single-photon emission computed tomography (SPECT), are used to quantify amyloid deposition *in vivo.* The term "*in vivo* imaging" refers to any method which permits the detection of a labeled thioflavin derivative as described herein. For gamma imaging, the radiation emitted from the organ or area being examined is measured and expressed either as total binding or as a ratio in which total binding in one tissue is normalized to (for example, divided by) the total binding in another tissue of the same subject during the same *in vivo* imaging procedure. Total binding *in vivo* is defined as the entire signal detected in a tissue by an *in vivo* imaging technique without the need for correction by a second injection of an identical quantity of labeled compound along with a large excess of unlabeled, but otherwise chemically identical compound. A "subject" is a mammal, preferably a human, and most preferably a human suspected of having dementia.

For purposes of *in vivo* imaging, the type of detection instrument available is a major factor in selecting a given label. For instance, radioactive isotopes and ¹⁹F are particularly suitable for *in vivo* imaging in the methods of the present invention. The type of instrument used will guide the selection of the radionuclide or stable isotope. For instance, the radionuclide chosen must have a type of decay detectable by a given type of instrument. Another consideration relates to the half-life of the radionuclide. The half-life should be long enough so that it is still detectable at the time of maximum uptake by the target, but short enough so that the host does not sustain deleterious radiation. The radiolabeled compounds of the invention can be detected using gamma imaging wherein emitted gamma irradiation of the appropriate wavelength is detected. Methods of gamma imaging include, but are not limited to, SPECT and PET. Preferably, for SPECT detection, the chosen radiolabel will lack a particulate emission, but will produce a large number of photons in a 140-200 keV range. For PET detection, the radiolabel will be a positron-emitting radionuclide such as ¹⁹F which will annihilate to form two 511 keV gamma rays which will be detected by the PET camera.

In the present invention, amyloid binding compounds/probes are made which are useful for *in vivo* imaging and quantification of amyloid deposition. These compounds are to be used in conjunction with non-invasive neuroimaging techniques such as magnetic resonance spectroscopy (MRS) or imaging (MRI), positron emission tomography (PET), and single-photon emission computed tomography (SPECT). In accordance with this invention, the thioflavin derivatives may be labeled with ¹⁹F or ¹³C for MRS/MRI by general organic chemistry techniques known to the art. For example, see ADVANCED ORGANIC CHEMISTRY: REACTION, MECHANISMS, AND STRUCTURE, 3rd ed. (1985), the contents of which are hereby incorporated by reference. The thioflavin derivatives also may be radiolabeled with ¹⁸F, ¹¹C, ⁷⁵Br, or ⁷⁶Br for PET by techniques well known in the art and are described by Fowler, J. and Wolf, A. in POSITRON EMISSION TOMOGRAPHY AND AUTORADIOGRAPHY 391-450 (Raven Press, 1986), the contents of which are hereby incorporated by reference. The thioflavin derivatives also may be radiolabeled with ¹²³I for SPECT by any of several techniques known to the art. *See, e.g.,* Kulkarni, Int. J. Rad. Appl. & Inst. (Part B) 18: 647 (1991), the contents of which are hereby incorporated by reference. In addition, the thioflavin derivatives may be labeled with any suitable radioactive iodine isotope, such as, but not limited to ¹³¹I, ¹²⁵I, or ¹²³I, by iodination of a diazotized amino derivative directly via a diazonium iodide, *see* Greenbaum, F. Am. J. Pharm. 108: 17 (1936), or by conversion of the unstable diazotized amine to the stable triazene, or by conversion of a non-radioactive halogenated precursor to a stable tri-alkyl tin derivative which then can be converted to the iodo compound by several methods well known to the art. See, Satyamurthy and Barrio, J. Org. Chem. 48: 4394 (1983), Goodman et al., J. Org. Chem. 49: 2322 (1984), and Mathis et al., J. Labell. Comp. and Radiopharm. 1994: 905; Chumpradit et al., J. Med. Chem. 34: 877 (1991); Zhuang et al., J. Med. Chem. 37: 1406 (1994); Chumpradit et al., J. Med. Chem. 37: 4245 (1994). For example, a stable triazene or tri-alkyl tin derivative of thioflavin or its analogues is reacted with a halogenating agent containing ¹³¹I, ¹²⁵I, ¹²³I, ⁷⁶Br, ⁷⁵Br, ¹⁸F or ¹⁹F. Thus, the stable tri-alkyl tin derivatives of thioflavin and its analogues are novel precursors useful for the synthesis of many of the radiolabeled compounds within the present invention. As such, these tri-alkyl tin derivatives are one embodiment of this invention.

The thioflavin derivatives also may be radiolabeled with known metal radiolabels, such as Technetium-99m (^{99m}Tc). Modification of the substituents to introduce ligands that bind such metal ions can be effected without undue experimentation by one of ordinary skill in the radiolabeling art. The metal radiolabeled thioflavin derivative can then be used to detect amyloid deposits. Preparing radiolabeled derivatives of Tc^{99m} is well known in the art. See, for example, Zhuang et al., "Neutral and stereospecific Tc-99m complexes: [99mTc]N-benzyl-3,4-di-(N-2-mercaptoethyl)-amino-pyrrolidines (P-BAT)" Nuclear Medicine & Biology 26(2):217-24, (1999); Oya et al., "Small and neutral Tc(v)O BAT, bisaminoethanethiol (N2S2) complexes for developing new brain imaging agents" Nuclear Medicine & Biology 25(2):135-40, (1998); and Hom et al., "Technetium-99m-labeled receptor-specific small-molecule radiopharmaceuticals: recent developments and encouraging results" Nuclear Medicine & Biology 24(6):485-98, (1997).

The methods of the present invention may use isotopes detectable by nuclear magnetic resonance spectroscopy for purposes of *in vivo* imaging and spectroscopy. Elements particularly useful in magnetic resonance spectroscopy include ¹⁹F and ¹³C.

Suitable radioisotopes for purposes of this invention include beta-emitters, gammaemitters, positron-emitters, and x-ray emitters. These radioisotopes include ¹³¹I, ¹²³I, ¹⁸F, ¹¹C, ⁷⁵Br, and ⁷⁶Br. Suitable stable isotopes for use in Magnetic Resonance Imaging (MRI) or Spectroscopy (MRS), according to this invention, include ¹⁹F and ¹³C. Suitable radioisotopes for *in vitro* quantification of amyloid in homogenates of biopsy or post-mortem tissue include ¹²⁵I, ¹⁴C, and ³H. The preferred radiolabels are ¹¹C or ¹⁸F for use in PET *in vivo* imaging, ¹²³I for use in SPECT imaging, ¹⁹F for MRS/MRI, and ³H or ¹⁴C for *in vitro* studies. However, any conventional method for visualizing diagnostic probes can be utilized in accordance with this invention.

According to the aspect of the invention which relates to a method of detecting amyloid deposits in biopsy or post-mortem tissue, the method involves incubating formalin-fixed tissue with a solution of a thioflavin amyloid binding compound of the present invention. Preferably, the solution is 25-100% ethanol, (with the remainder being water) saturated with a thioflavin amyloid binding compound according to the present invention. Upon incubation, the compound stains or labels the amyloid deposit in the tissue, and the stained or labeled deposit can be detected or visualized by any standard method. Such detection means include microscopic techniques such as bright-field, fluorescence, laser-confocal and cross-polarization microscopy.

The method of quantifying the amount of amyloid in biopsy or post-mortem tissue involves incubating a labeled derivative of thioflavin according to the present invention, or a water-soluble, non-toxic salt thereof, with homogenate of biopsy or post-mortem tissue. The tissue is obtained and homogenized by methods well known in the art. The preferred label is a radiolabel, although other labels such as enzymes, chemiluminescent and immunofluorescent compounds are well known to skilled artisans. The preferred radiolabel is ¹²¹I, ¹⁴C or ³H which is contained in a substituent substituted on one of the compounds of the present formulae described herein. Tissue containing amyloid deposits will bind to the labeled derivatives of the thioflavin amyloid binding compounds of the present invention. The bound tissue is then separated from the unbound tissue by any mechanism known to the skilled artisan, such as filtering. The bound tissue can then be quantified through any means known to the skilled artisan. The units of tissue-bound radiolabeled thioflavin derivative are then converted to units of micrograms of amyloid per 100 mg of tissue by comparison to a standard curve generated by incubating known amounts of amyloid with the radiolabeled thioflavin derivative.

The method of distinguishing an Alzheimer's diseased brain from a normal brain involves obtaining tissue from (a) the cerebellum and (b) another area of the same brain, other than the cerebellum, from normal subjects and from subjects suspected of having Alzheimer's disease. Such tissues are made into separate homogenates using methods well known to the skilled artisan, and then are incubated with a radiolabeled thioflavin amyloid binding compound. The amount of tissue which binds to the radiolabeled thioflavin amyloid binding compound is then calculated for each tissue type (e.g. cerebellum, non-cerebellum, normal, abnormal) and the ratio for the binding of non-cerebellum to cerebellum tissue is calculated for tissue from normal and for tissue from patients suspected of having Alzheimer's disease. These ratios are then compared. If the ratio from the brain suspected of having Alzheimer's disease is above 90% of the ratios obtained from normal brains, the diagnosis of Alzheimer's disease is made. The normal ratios can be obtained from previously obtained data, or alternatively, can be recalculated at the same time the suspected brain tissue is studied.

The ability of the present compounds to specifically bind to neurofibrially tangles over amyloid plaques is particularly true at concentrations less than 10 nM, which includes the in vivo concentration range of PET radiotraces. At these low concentrations, which contains only tangles and no plaques, significant binding does not result when compared to control brain tissue containing neither plaques nor tangles. However, incubation of homogenates of brain tissue which contains mainly plaques and some tangles with radiolabeled compounds of the formulae described herein, results in a significant increase in binding when compared to control tissue without plaques or tangles. This data suggests the advantage that these compounds are specific for Aβ deposits at concentrations less than 10 nM. These low concentrations are detectable in PET studies, making PET detection using radiolabeled compounds of the formulae herein described which are specific for Aβ deposits possible. The use of such compounds permits PET detection in Aβ deposits such as those found in plaques and cerebrovascular amyloid. Since it has been reported that Aβ levels in the frontal cortex are increased prior to tangle formation, this would suggest that radiolabeled compounds of the present invention, used as PET tracers, would be specific for the earliest changes in AD cortex. Naslund et al., JAMA 283: 1571 (2000).

### Method for Detecting Amyloid Deposit(s) In Vivo

This invention further provides a method for detecting amyloid deposit(s) *in vivo,* comprising:
(i) administering to an animal an effective amount of an inventive compound, wherein the compound would bind to any amyloid deposit(s) in the animal; and
(ii) detecting binding of the compound to amyloid deposit(s) in the animal.

After a sufficient time has elapsed for the compound to bind with the amyloid deposit(s), for example 30 minutes to 48 hours following administration, the binding may be detected by any means known in the art. Examples of detection means include, without limitation, assays (such as immunometric, calorimetric, densitometric, spectrographic and chromatographic assays), non-invasive neuroimaging techniques (such as magnetic resonance spectroscopy (MRS), magnetic resonance imaging (MRI), and gamma imaging techniques such as single-photon emission computed tomography (SPECT) and positron emission tomography (PET). For gamma imaging, the radiation emitted from the organ or area being examined is measured and expressed either as total binding or as a ratio in which total binding in one tissue is normalized to (for example, divided by) the total binding in another tissue of the same subject during the same *in vivo* imaging procedure. Total binding *in vivo* is defined as the entire signal detected in a tissue by an *in vivo* imaging technique without the need for correction by a second injection of an identical quantity of labeled compound along with a large excess of unlabeled, but otherwise chemically identical compound.

The type of detection instrument available may be a factor in selecting the radioactive halo or carbon isotope. For instance, the selected radioisotope should have a type of decay that is detectable by a given instrument. Another consideration relates to the half-life of the radioisotope. The half-life should be long enough such that the radioisotope is still detectable at the time of maximum uptake by the target, but short enough such that the host does not sustain deleterious radiation. For SPECT detection, the selected radioisotope may lack a particulate emission, but may produce a large number of photons in the 140-200 keV range. For PET detection, the selected radioisotope may be a positron-emitting radioisotope, which annihilates to form two 511 keV gamma rays detectable by a PET camera.

Useful radioisotopes include, without limitation: ¹²⁵I, ¹⁴C, and ³H for *in vitro* quantification of amyloid in homogenates of biopsy or post-mortem tissue; ¹¹C and ¹⁸F for PET *in vivo* imaging; ¹²³I for SPECT imaging; ¹⁸F for MRS/MRI; ³H or ¹⁴C for *in vitro* studies; and ¹⁸F and ¹³C for magnetic resonance spectroscopy. In one embodiment, the detecting is effected by gamma imaging, magnetic resonance imaging or magnetic resonance spectroscopy. In another embodiment, the gamma imaging is PET or SPECT.

The inventive compound may be administered by any means known to one of ordinary skill in the art. For example, administration to the animal may be local or systemic and accomplished orally, parenterally, by inhalation spray, topically, rectally, nasally, buccally, vaginally, or via an implanted reservoir. The term "parenteral" as used herein includes subcutaneous, intravenous, intraarterial, intramuscular, intraperitoneal, intrathecal, intraventricular, intrasternal, intracranial, and intraosseous injection and infusion techniques. The exact administration protocol will vary depending upon various factors including the age, body weight, general health, sex and diet of the patient; the determination of specific administration procedures would be routine to an one of ordinary skill in the art.

Dose levels on the order of about 0.001 µg/kg/day to about 10,000 mg/kg/day of an inventive compound are useful for the inventive methods. In one embodiment, the dose level is about 0.001 µg/kg/day to about 10 µg/kg/day. In another embodiment, the dose level is about 0.01 µg/kg/day to about 1.0 µg/kg/day. In yet another embodiment, the dose level is about 0.1 mg/kg/day to about 100 mg/kg/day.

The specific dose level for any particular patient will vary depending upon various factors, including the activity and the possible toxicity of the specific compound employed; the age, body weight, general health, sex and diet of the patient; the time of administration; the rate of excretion; the drug combination; and the form of administration. Typically, *in vitro* dosage-effect results provide useful guidance on the proper doses for patient administration. Studies in animal models are also helpful. The considerations for determining the proper dose levels are well known in the art and within the skills of an ordinary physician.

Any known administration regimen for regulating the timing and sequence of drug delivery may be used and repeated as necessary to effect treatment in the inventive methods. The regimen may include pretreatment and/or co-administration with additional therapeutic agent(s).

In one embodiment, the inventive compounds are administered to an animal that is suspected of having or that is at risk of developing a disease, disorder or condition characterized by amyloid deposition. For example, the animal may be an elderly human.

In another embodiment, the inventive compounds bind to Aβ with a dissociation constant (K_{D}) of about 0.0001 µM to about 10.0 µM when measured by binding to synthetic Aβ peptide or AD brain tissue.

### Method for Detecting Amyloid Deposit(s) In Vitro

This invention further provides a method for detecting amyloid deposit(s) *in vitro* comprising:
(i) contacting a bodily tissue with an effective amount of an inventive compound, wherein the compound would bind any amyloid deposit(s) in the tissue; and
(ii) detecting binding of the compound to amyloid deposit(s) in the tissue.

The binding may be detected by any means known in the art. Examples of detection means include, without limitation, microscopic techniques, such as bright-field, fluorescence, laser-confocal and cross-polarization microscopy.

In one embodiment, the tissue is biopsy or post-mortem tissue that is formalin-fixed or fresh-frozen. In another embodiment, the tissue is homogenized. In yet another embodiment, the inventive compound is in a solution that further comprises 25-99% ethanol, with the remainder of the solution being water. In yet another embodiment, the solution comprises 0-50% ethanol and 0.0001 to 100 µM of the compound. In yet another embodiment, the method further comprises (iii) separating from the tissue the amyloid deposit(s) bound to the compound; and (iv) quantifying the amyloid deposit(s) bound to the inventive compound. The bound amyloid deposit(s) may be separated from the tissue by any means known in the art, such as filtering. The amount of bound amyloid deposit(s) may be converted to units of µg of amyloid deposit(s) per 100 mg of tissue by comparison to a standard curve generated by incubating known amounts of amyloid with the inventive compound or pharmaceutically acceptable salt, hydrate, solvate or prodrug.

### Method for Distinguishing Alzheimer's Diseased Brain from Normal Brain

This invention further provides a method for distinguishing an Alzheimer's diseased brain from a normal brain comprising:
(i) obtaining tissues from (a) the cerebellum and (b) another area of the same brain, of a normal animal and of an animal suspected of having Alzheimer's disease;
(ii) contacting the tissues with an inventive compound;
(iii) quantifying the amyloid bound to the compound;
(iv) calculating the ratio of the amount of amyloid in the area of the brain other than the cerebellum to the amount of amyloid in the cerebellum;
(v) comparing the ratio for a normal animal with the ratio for an animal suspected of having Alzheimer's disease.

A diagnosis of Alzheimer's disease may be made if the ratio for an animal suspected of having Alzheimer's disease is, for example, above 90% of the ratio for a normal animal. For this method, a "normal" animal is one that is not suffering from Alzheimer's disease.

### PHARMACEUTICAL COMPOSITION

This invention further provides a pharmaceutical composition comprising:
(i) an effective amount of at least one inventive compound; and
(ii) a pharmaceutically acceptable carrier.

The composition may comprise one or more additional pharmaceutically acceptable ingredient(s), including without limitation one or more wetting agent(s), buffering agent(s), suspending agent(s), lubricating agent(s), emulsifier(s), disintegrant(s), absorbent(s), preservative(s), surfactant(s), colorant(s), flavorant(s), sweetener(s) and therapeutic agent(s).

The composition may be formulated into solid, liquid, gel or suspension form for: (1) oral administration as, for example, a drench (aqueous or non-aqueous solution or suspension), tablet (for example, targeted for buccal, sublingual or systemic absorption), bolus, powder, granule, paste for application to the tongue, hard gelatin capsule, soft gelatin capsule, mouth spray, emulsion and microemulsion; (2) parenteral administration by subcutaneous, intramuscular, intravenous or epidural injection as, for example, a sterile solution, suspension or sustained-release formulation; (3) topical application as, for example, a cream, ointment, controlled-release patch or spray applied to the skin; (4) intravaginal or intrarectal administration as, for example, a pessary, cream or foam; (5) sublingual administration; (6) ocular administration; (7) transdermal administration; or (8) nasal administration.

In one embodiment, the composition is formulated for intravenous administration and the carrier includes a fluid and/or a nutrient replenisher. In another embodiment, the composition is capable of binding specifically to amyloid *in vivo,* is capable of crossing the blood-brain barrier, is non-toxic at appropriate dose levels and/or has a satisfactory duration of effect. In yet another embodiment, the composition comprises about 10 mg of human serum albumin and from about 0.5 to 500 mg of the inventive compound per milliliter of phosphate buffer containing NaCl.

The following examples are given to illustrate the present invention. It should be understood, however, that the invention is not to be limited to the specific conditions or details described in these examples. Throughout the specification, any and all references to a publicly available document, including U.S. patents, are specifically incorporated into this patent application by reference.

### EXAMPLES

All of the reagents used in the synthesis were purchased from Aldrich Chemical Company and used without further purification, unless otherwise indicated. Melting points were determined on Mel-TEMP II and were uncorrected. The ¹H NMR spectra of all compounds were measured on Bruker 300 using TMS as internal reference and were in agreement with the assigned structures. The TLC was performed using Silica Gel 60 F₂₅₄ from EM Sciences and detected under UV lamp. Flash chromatography was performed on silica gel 60 (230-400 mesh. Purchased from Mallinckrodt Company. The reverse phase TLC were purchased from Whiteman Company.

### General Methodology for Synthesis of Compound of Formula I:

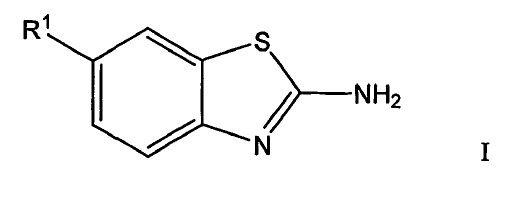

R¹ is hydrogen, -OH, -NO₂, -CN, -COOR, -OCH₂OR, C₁-C₆ alkyl, C₂-C₆ alkenyl, C₂-C₆ alkynyl, C₁-C₆ alkoxy or halo, wherein one or more of the atoms of R¹ may be a radiolabeled atom;
R is C₁-C₆ alkyl, wherein one or more of the carbon atoms may be a radiolabeled atom;
is hydrolysed by one of the following two procedures:

### Preparation of 2-aminothiophenol via hydrolysis:

The 6-substituted 2-aminobenzothiazole (172 mmol) is suspended in 50% KOH (180 g KOH dissolved in 180 mL water) and ethylene glycol (40 mL). The suspension is heated to reflux for 48 hours. Upon cooling to room temperature, toluene (300 mL) is added and the reaction mixture is neutralized with acetic acid (180 mL). The organic layer is separated and the aqueous layer is extracted with another 200 mL of toluene. The toluene layers are combined and washed with water and dried over MgSO₄. Evaporation of the solvent gives the desired product.

### Preparation of 2-aminothiophenol via hydrazinolysis:

The 6-substituted benzothiazole (6.7mmol)) is suspended in ethanol (11 mL, anhydrous) and hydrazine (2.4 mL) is added under a nitrogen atmosphere at room temperature. The reaction mixture is heated to reflux for 1 hour. The solvent is evaporated and the residue is dissolved into water (10mL) and adjusted to a pH of 5 with acetic acid. The precipitate is collected with filtration and washed with water to give the desired product.

The resulting 5-substituted-2-amino-1-thiophenol of the form 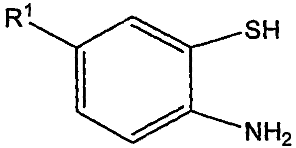 is coupled to a benzoic acid of the form: 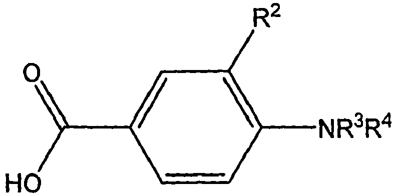 wherein R² is hydrogen, and R³ and R⁴ are independently hydrogen, C₁-C₆ alkyl, C₂-C₆ alkenyl or C₂-C₆ alkynyl
by the following reaction:

A mixture of the 5-substituted 2-aminothiophenol (4.0 mmol), the benzoic acid (4.0 mmol), and polyphosphoric acid (PPA) (10 g) is heated to 220° C for 4 hours. The reaction mixture is cooled to room temperature and poured into 10% potassium carbonate solution (~400 mL). The precipitate is collected by filtration under reduced pressure to give the desired product, which can be purified by flash chromatography or recrystallization.

The R² hydrogen can be substituted with either a non-radioactive halo or a radioactive halo by the following reaction:
To a solution of 6-substituted 2-(4'-aminophenyl)-benzothiazole (1 mg) in 250 µL acetic acid in a sealed vial is added 40, µL of chloramine-T solution (28 mg dissolved in 500 µL acetic acid) followed by 27 µL (ca. 5 mCi) of sodium [¹²⁵I]iodide (specific activity 2,175 Ci/mmol). The reaction mixture is stirred at room temperature for 2.5 hours and quenched with saturated sodium hydrogensulfite solution. After dilution with 20 ml of water, the reaction mixture is loaded onto C8 Plus SepPak and eluted with 2 ml methanol. Depending on the nature of the substituent on the 6-position, protecting groups may need to be employed. For example, the 6-hydroxy group is protected as the methanesulfonyl (mesyloxy) derivative. For deprotection of the methanesulfonyl group, 0.5 ml of 1 M NaOH is added to the eluted solution of radioiodinated intermediate. The mixture is heated at 50 °C for 2 hours. After being quenched by 500 µL of 1 M acetic acid, the reaction mixture is diluted with 40 mL of water and loaded onto a C8 Plus SepPak. The radioiodinated product, having a radioactivity of ca. 3 mCi, is eluted off the SepPak with 2 mL of methanol. The solution is condensed by a nitrogen stream to 300 µL and the crude product is purified by HPLC on a Phenomenex ODS column (MeCN/TEA buffer, 35:65, pH 7.5, flow rate 0.5 mL/minute up to 4 minutes, 1.0 mL/minute at 4-6 minutes, and 2.0 mL/minute after 6 minutes, retention time 23.6). The collected fractions are loaded onto a C8 Plus SepPak. Elution with 1 mL of ethanol gave ca. 1 mCi of the final radioiodinated product.

When either or both R³ and R⁴ are hydrogen, then R³ and R⁴ can be converted to C₁-C₆ alkyl, C₂-C₆ alkenyl or C₂-C₆ alkynyl by reaction with an alkyl, alkenyl or alkynyl halide under the following conditions:

For dialkylation: To a solution of 6-substituted 2-(4'-aminophenyl)-benzothiazole (0.59 mmol) in DMSO (anhydrous, 2 ml) are added alkyl, alkenyl, or alkynyl halide (2.09 mmol), and K₂CO₃ (500 mg, 3.75 mmol). The reaction mixture is heated at 140°C for 16 hours. Upon cooling to room temperature, the reaction mixture is poured into water and extracted with ethyl acetate (3 x 10 mL). The organic layers are combined and the solvent is evaporated. The residue is purified by flash column to give the desired 6-substituted dimethylaminophenyl)-benzothiazole.

For monoalkylation: To a solution of 6-substituted 2-(4'-aminophenyl)-benzothiazole (0.013 mmol) in DMSO (anhydrous, 0.5 ml) is added alkyl, alkenyl, or alkynyl halide (0.027 mmol) and anhydrous K₂CO₃ (100 mg, 0.75 mmol). The reaction mixture is heated at 100°C for 16 hours. Upon cooling to room temperature, the reaction mixture is directly purified by normal phase preparative TLC to give the desired 6-substituted-2-(4'-methylaminophenyl)-benzothiazole derivatives.

When R² is hydrogen or a non-radioactive halo, R⁴ is C₁-C₆ alkyl, C₂-C₆ alkenyl or C₂-C₆ alkynyl, wherein the alkyl, alkenyl or alkynyl comprises a radioactive carbon or is substituted with a radioactive halo, the compound can be synthesized by one of the following sequences:

### For radioactive carbon incorporation:

Approximately 1 Ci of [¹¹C]carbon dioxide is produced using a CTI/Siemens RDS 112 negative ion cyclotron by irradiation of a nitrogen gas (¹⁴N₂) target containing 1% oxygen gas with a 40 µA beam current of 11 MeV protons for 60 minutes. [¹¹C] Carbon dioxide is converted-to [¹¹C]methyl iodide by first reacting it with a saturated solution of lithium aluminum hydride in THF followed by the addition of hydriodic acid at reflux temperature to generate [¹¹C]methyl iodide. The [¹¹C]methyl iodide is carried in a stream of nitrogen gas to a reaction vial containing the precursor for radiolabeling. The precursor, 6-substituted 2-(4'-aminophenyl)-benzothiazole (~3.7 µmoles), is dissolved in 400 µL of DMSO. Dry KOH (10 mg) is added, and the 3 mL V-vial is vortexed for 5 minutes. No-carrier-added [¹¹C]methyl iodide is bubbled through the solution at 30 mL/minute at room temperature. The reaction is heated for 5 minutes at 95° C using an oil bath. The reaction product is purified by semi-preparative HPLC using a Prodigy ODS-Prep column eluted with 60% acetonitrile/40% triethylammonium phosphate buffer pH 7.2 (flow at 5 mL/minute for 0-7 minutes then increased to 15 mL/minute for 7-30 minutes). The fraction containing [N-methyl-¹¹C] 6-substituted 2-(4'-methylaminophenyl)-benzothiazole (at about 15 min) is collected and diluted with 50 mL of water and eluted through a Waters C18 SepPak Plus cartridge. The C18 SepPak is washed with 10 mL of water, and the product is eluted with 1 mL of ethanol (absolute) into a sterile vial followed by 14 mL of saline. Radiochemical and chemical purities are >95% as determined by analytical HPLC (k' = 4.4 using the Prodigy ODS(3) analytical column eluted with 65/35 acetonitrile/triethylammonium phosphate buffer pH 7.2). The radiochemical yield averages 17% at EOS based on [¹¹C]methyl iodide, and the specific activity averages about 160 GBq/µmol (4.3 Ci/µmol) at end of synthesis.

### For radioactive halogen incorporation:

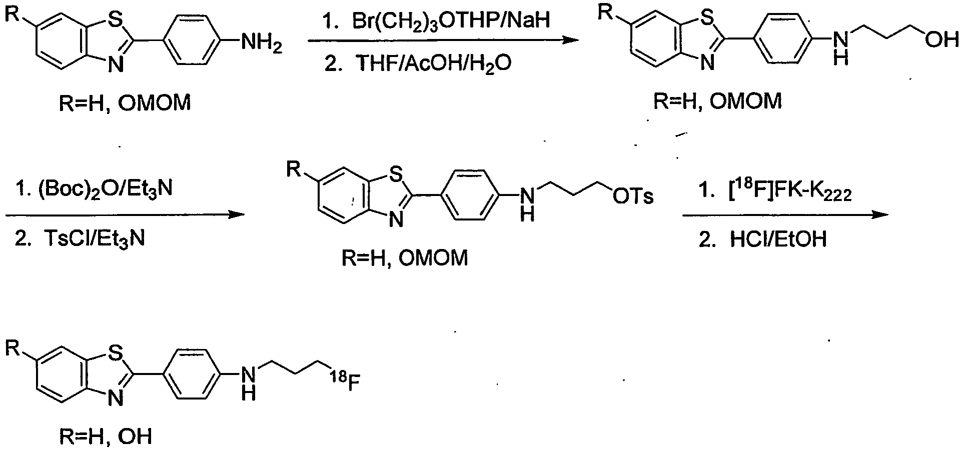

A mixture of 6-substituted 2-(4'-aminophenyl)-benzathiazole (protecting groups may be necessary depending on the nature of the 6-substituent as noted above) (0.22 mmol), NaH (4.2 mmol) and 2-(-3-bromopropoxy)tetrahydro-2-H-pyran (0.22 mmol) in THF (8 mL) is heated to reflux for 23 hours. The solvent is removed by distillation and the residue is dissolved in to ethyl acetate and water, the organic layer is separated and the aqueous layer is extracted with ethyl acetate (10 mL x 6). The organic layer is combined and dried over MgSO₄ and evaporated to dryness. The residue is added AcOH/THF/H₂O solution (5 mL, 4/2/1) and heated to 100°C for 4 hours. The solvent is removed by evaporation and the residue is dissolved in ethyl acetate (~10 mL) washed by NaHCO₃ solution, dried over MgSO₄ and evaporated to dryness to give a residue which is purified with preparative TLC(hexane:ethyl acetate=60:40) to give the desired 6-substituted 2-(4'-(3"-hydroxypropylamino)-phenyl)-benzothiazole (45%).

To a solution of 6-substituted 2-(4'-(3"-hydroxypropylamino)-phenyl)-benzathiazole(0.052 mmol) and Et₃N(0.5 ml) dissolved in acetone (5 mL) is added (Boc)₂O (50 mg, 0.22 mmol). The reaction mixture is stirred at room temperature for 6 hours followed by addition of tosyl chloride (20 mg, 0.11 mmol). The reaction mixture is stirred at room temperature for another 24 hours. The solvent is removed and the residue is dissolved into ethyl acetate (10 mL), washed with NaCO₃ solution, dried over MgSO₄, evaporated, and purified with flash column (Hexane/ethyl acetate=4/1) to give the desired 6-substituted 2-(4'-(3"-toluenesulfonoxypropylamino)-phenyl)-benzothiazole (13%). This 6-substituted 2-(4'-(3"-toluenesulfonoxypropylamino)-phenyl)-benzothiazole is then radiofluorinated by standard methods as follows:
A cyclotron target containing 0.35 mL of 95% [O-18]-enriched water is irradiated with 11 MeV protons at 20µA of beam current for 60 minutes, and the contents are transferred to a 5 mL reaction vial containing Kryptofix 222 (22.3 mg) and K₂CO₃ (7.9 mg) in acetonitrile (57µL). The solution is evaporated to dryness three times at 110°C under a stream of argon following the addition of 1 mL aliquots of acetonitrile. To the dried [F-18]fluoride is added 3 mg of 6-substituted 2-(4'-(3"-toluenesulfonoxypropylamino)-phenyl)-benzothiazole in 1mL DMSO, and the reaction vial is sealed and heated to 85°C for 30 minutes. To the reaction vial, 0.5 mL of MeOH/HCl (concentrated) (2/1 v/v) is added, and the vial is heated at 120°C for 10 minutes. After heating, 0.3 mL of 2 M sodium acetate buffer is added to the reaction solution followed by purification by semi-prep HPLC using a Phenomenex Prodigy ODS-prep C18 column (10µm 250x10 mm) eluted with 40% acetonitrile/ 60% 60 mM triethylamine-phosphate buffer (v/v) pH 7.2 at a flow rate of 5 mL/minute for 15 minutes, then the flow is increased to 8 mL/minute for the remainder of the separation. The product, [F-18]6-substituted 2-(4'-(3"-fluoropropylamino)-phenyl)-benzothiazole, is eluted at ~20 minutes in a volume of about 16 mL. The fraction containing [F-18]6-substituted 2-(4'-(3"-fluoropropylamino)-phenyl)-benzothiazole is diluted with 50 mL of water and eluted through a Waters C18 SepPak Plus cartridge. The SepPak cartridge is then washed with 10 mL of water, and the product is eluted using 1 mL of ethanol (absol.) into a sterile vial. The solution is diluted with 10 mL of sterile normal saline for intravenous injection into animals. The [F-18]6-substituted 2-(4'-(3"-fluoropropylamino)-phenyl)-benzothiazole product is obtained in 2-12% radiochemical yield at the end of the 120 minute radiosynthesis (not decay corrected) with an average specific activity of 1500 Ci/mmol.

### Synthesis Examples

### Ref Example 1: [N-Methyl-¹¹C]2-(4'-Dimethylaminophenyl)-6-methoxy-benzothiazole was synthesized according to Scheme I.

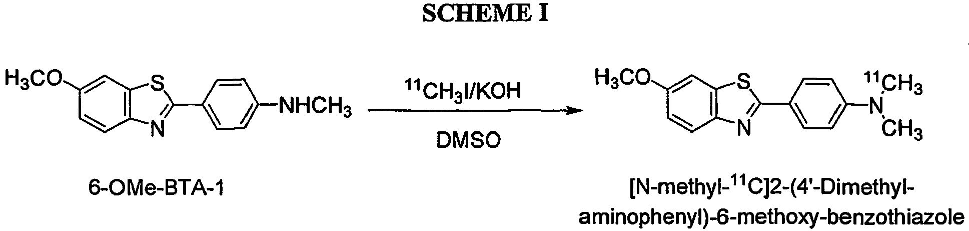

Approximately 1 Ci of [¹¹C]carbon dioxide was produced using a CTI/Siemens RDS 112 negative ion cyclotron by irradiation of a nitrogen gas (¹⁴N₂) target containing 1% oxygen gas with a 40 µA beam current of 11 MeV protons for 60 minutes. [¹¹C]Carbon dioxide is converted to [¹¹C]methyl iodide by first reacting it with a saturated solution of lithium aluminum hydride in THF followed by the addition of hydriodic acid at reflux temperature to generate [¹¹C]methyl iodide. The [¹¹C]methyl iodide is carried in stream of nitrogen gas to a reaction vial containing the precursor for radiolabeling. The precursor, 6-CH₃O-BTA-1 (1.0 mg, 3.7 µmoles), was dissolved in 400 µL of DMSO. Dry KOH (10 mg) was added, and the 3 mL V-vial was vortexed for 5 minutes. No-carrier-added [¹¹C]methyl iodide was bubbled through the solution at 30 mL/minute at room temperature. The reaction was heated for 5 minutes at 95°C using an oil bath. The reaction product was purified by semi-preparative HPLC using a Prodigy ODS-Prep column eluted with 60% acetonitrile/40% triethylammonium phosphate buffer pH 7.2 (flow at 5 mL/minute for 0-7 minutes then increased to 15 mL/minute for 7-30 minutes). The fraction containing [N-Methyl-¹¹C]2-(4'-Dimethylaminophenyl)-6-methoxy-benzothiazole (at about 15 minutes) was collected and diluted with 50 mL of water and eluted through a Waters C18 SepPak Plus cartridge. The C18 SepPak was washed with 10 mL of water, and the product was eluted with 1 mL of ethanol (absolute) into a sterile vial followed by 14 mL of saline. Radiochemical and chemical purities were >95% as determined by analytical HPLC (k' = 4.4 using the Prodigy ODS(3) analytical column eluted with 65/35 acetonitrile/triethylammonium phosphate buffer pH 7.2). The radiochemical yield averaged 17% at EOS based on [¹¹C]methyl iodide, and the specific activity averaged about 160 GBq/µmol (4.3 Ci/µmol) at end of synthesis.

### Ref Example 2: 2-(3'-¹²⁵I-iodo-4'-amino-phenyl)-benzothiazol-6-ol was synthesized according to Scheme II.

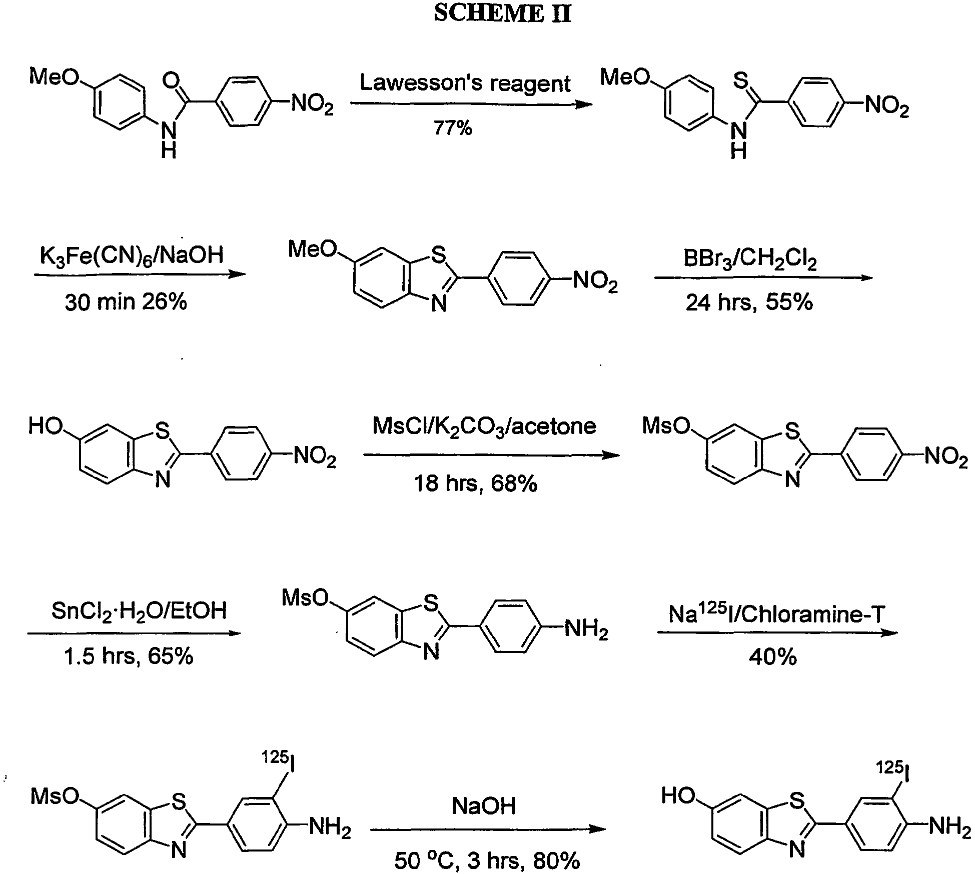

To a solution of 2-(4'-aminophenyl)-6-methanesulfonoxy-benzothiazole (1 mg) in 250 µL acetic acid in a sealed vial was added 40 µL of chloramine T solution (28 mg dissolved in 500 µL acetic acid) followed by 27 µL (ca. 5 mCi) of sodium [¹²⁵I]iodide (specific activity 2,175 Ci/mmol). The reaction mixture was stirred at room temperature for 2.5 hours and quenched with saturated sodium hydrogensulfite solution. After dilution with 20 ml of water, the reaction mixture was loaded onto C8 Plus SepPak and eluted with 2 ml methanol. For deprotection of the methanesulfonyl group, 0.5 ml of 1 M NaOH was added to the eluted solution of radioiodinated intermediate. The mixture was heated at 50°C for 2 hours. After being quenched by 500 µL of 1 M acetic acid, the reaction mixture was diluted with 40 mL of water and loaded onto a C8 Plus SepPak. The radioiodinated product, having a radioactivity of ca. 3 mCi, was eluted off the SepPak with 2 mL of methanol. The solution was condensed by a nitrogen stream to 300 µL and the crude product was purified by HPLC on a Phenomenex ODS column (MeCN/TEA buffer, 35:65, pH 7.5, flow rate 0.5 mL/minute up to 4 minutes, 1.0 mL/minute at 4-6 minutes, and 2.0 mL/minute after 6 minutes, retention time 23.6). The collected fractions were loaded onto a C8 Plus SepPak. Elution with 1 mL of ethanol gave ca. 1 mCi of the final radioiodinated product.

Preparation of the ¹²³I radiolabeled derivatives, proceeds similarly to the synthesis outlined above. For example, replacing sodium [¹²⁵I]iodide with sodium [¹²³I]iodide in the synthetic method would provide the ¹²³I radiolabeled compound. Such substitution of one radiohalo atom for another is well known in the art, see for example, Mathis CA, Taylor SE, Biegon A, Enas JD. [125I]5-Iodo-6-nitroquipazine: a potent and selective ligand for the 5-hydroxytryptamine uptake complex I. In vitro studies. Brain Research 1993; 619:229-235; Jagust W, Eberling JL, Roberts JA, Brennan KM, Hanrahan SM, Van Brocklin H, Biegon A, Mathis CA. In vivo imaging of the 5-hydroxytryptamine reuptake site in primate brain using SPECT and [123I]5-iodo-6-nitroquipazine. European Journal of Pharmacolgy 1993; 242:189-193; Jagust WJ, Eberling JL, Biegon A, Taylor SE, VanBrocklin H, Jordan S, Hanrahan SM, Roberts JA, Brennan KM, Mathis CA. [Iodine-123]5-Iodo-6-Nitroquipazine: SPECT Radiotracer to Image the Serotonin Transporter. Journal of Nuclear Medicine 1996; 37:1207-1214.)

### Example 3: 2-(3-¹⁸F-Fluoro-4-methylamino-phenyl)-benzothiazol-6-ol was synthesized according to Scheme III.

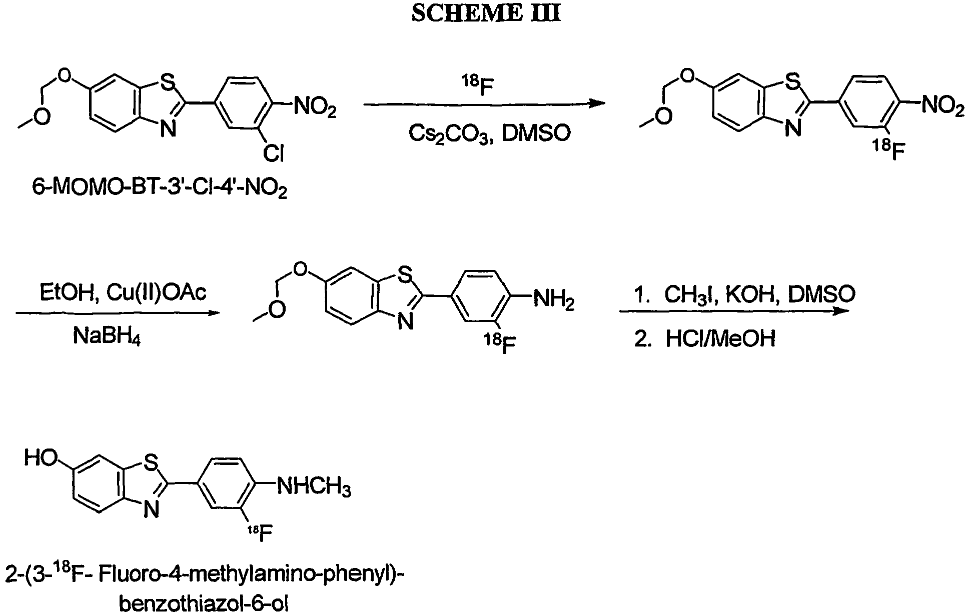

A cyclotron target containing 0.35 mL of 95% [O-18]-enriched water was irradiated with 11 MeV protons at 20 µA of beam current for 60 minutes, and the contents were transferred to a 5 mL reaction vial containing 2 mg Cs₂CO₃ in acetonitrile (57 µL). The solution was evaporated to dryness at 110°C under a stream of argon three times using 1 mL aliquots of acetonitrile. To the dried [F-18]fluoride was added 6 mg of 6-MOMO-BT-3'-Cl-4'-NO₂ in 1 mL DMSO, and the reaction vial was sealed and heated to 120°C for 20 minutes (radiochemical incorporation for this first radiosynthesis step was about 20% of solubilized [F-18]fluoride). To the crude reaction mixture was added 8 mL of water and 6 mL of diethyl ether, the mixture was shaken and allowed to separate. The ether phase was removed and evaporated to dryness under a stream of argon at 120°C. To the dried sample, 0.5 mL of absolute EtOH was added along with 3 mg copper (II) acetate and 8 mg of NaBH₄. The reduction reaction was allowed to proceed for 10 minutes at room temperature (the crude yield for the reduction step was about 40%). To the reaction mixture was added 8 mL of water and 6 mL of diethyl ether, the mixture was shaken and the ether phase separated. The diethyl ether phase was dried under a stream of argon at 120°C. To the reaction vial, 700 uL of DMSO was added containing 30 micromoles of CH₃I and 20 mg of dry KOH. The reaction vial was heated at 120°C for 10 minutes. A solution of 700 uL of 2:1 MeOH/HCl (concentrated) was added and heated for 15 minutes at 120°C. After heating, 1 mL of 2 M sodium acetate buffer was added to the reaction solution followed by purification by semi-prep HPLC using a Phenomenex Prodigy ODS-prep C18 column (10µm 250x10 mm) eluted with 35% acetonitrile/ 65% 60 mM triethylamine-phosphate buffer (v/v) pH 7.2 at a flow rate of 5 mL/minute for 2 minutes, then the flow was increased to 15 mL/minute for the remainder of the separation. The product, 2-(3-¹⁸F-fluoro-4-methylamino-phenyl)-benzothiazol-6-ol, eluted at ~15 minutes in a volume of about 16 mL. The fraction containing 2-(3-¹⁸F-fluoro-4-methylamino-phenyl)-benzothiazol-6-ol was diluted with 50 mL of water and eluted through a Waters C18 SepPak Plus cartridge. The SepPak cartridge was then washed with 10 mL of water, and the product was eluted using 1 mL of ethanol (absol.) into a sterile vial. The solution was diluted with 10 mL of sterile normal saline for intravenous injection into animals. The 2-(3-¹⁸F-fluoro-4-methylamino-phenyl)-benzothiazol-6-ol product was obtained in 0.5 % (n=4) radiochemical yield at the end of the 120 minute radiosynthesis (not decay corrected) with an average specific activity of 1000 Ci/mmol. The radiochemical and chemical purities of 2-(3-¹⁸F-fluoro-4-methylaminophenyl)-benzothiazol-6-ol were assessed by radio-HPLC with UV detection at 350 nm using a Phenomenex Prodigy ODS(3) C18 column (5 µm, 250 x 4.6 mm) eluted with 40% acetonitrile/ 60% 60 mM triethylamine-phosphate buffer (v/v) pH 7.2. 2-(3-¹⁸F-Fluoro-4-methylamino-phenyl)-benzothiazol-6-ol had a retention time of~11 minutes at a flow rate of 2 mL/min (k' = 5.5). The radiochemical purity was >99%, and the chemical purity was >90%. The radiochemical identity of 2-(3-¹⁸F-Fluoro-4-methylamino-phenyl)-benzothiazol-6-ol was confirmed by reverse phase radio-HPLC utilizing a quality control sample of the final radiochemical product co-injected with a authentic (cold) standard.

### Ref Example 4: 2-[4-(3-¹⁸F-Fluoro-propylamino)-phenyl]-benzothiazol-6-ol was synthesized according to Scheme IV.

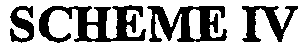

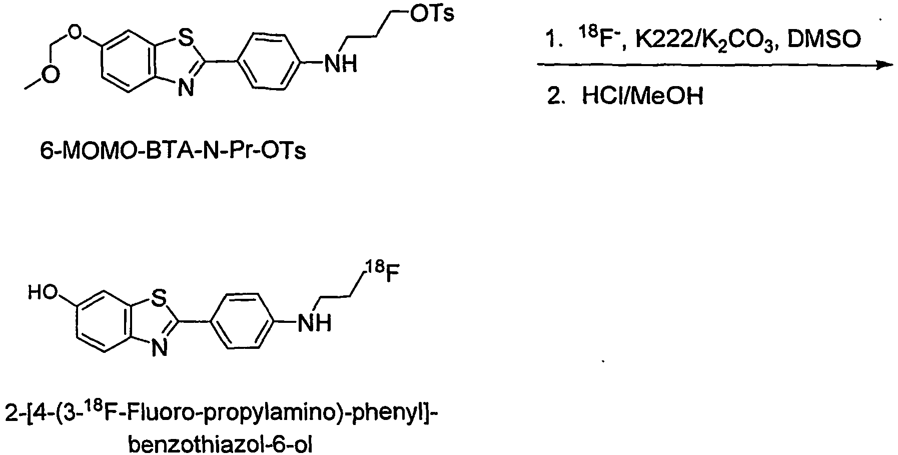

A cyclotron target containing 0.35 mL of 95% [O-18]-enriched water was irradiated with 11 MeV protons at 20 µA of beam current for 60 minutes, and the contents were transferred to a 5 mL reaction vial containing Kryptofix 222 (22.3 mg) and K₂CO₃ (7.9 mg) in acetonitrile (57 µL). The solution was evaporated to dryness three times at 110°C under a stream of argon following the addition of 1 mL aliquots of acetonitrile. To the dried [F-18]fluoride was added 3 mg of 6-MOMO-BTA-N-Pr-Ots in 1mL DMSO, and the reaction vial was sealed and heated to 85°C for 30 minutes. To the reaction vial, 0.5 mL of MeOH/HCl (concentrated) (2/1 v/v) was added, and the vial was heated at 120°C for 10 minutes. After heating, 0.3 mL of 2 M sodium acetate buffer was added to the reaction solution followed by purification by semi-prep HPLC using a Phenomenex Prodigy ODS-prep C18 column (10 µm 250x10 mm) eluted with 40% acetonitrile/ 60% 60 mM triethylamine-phosphate buffer (v/v) pH 7.2 at a flow rate of 5 mL/minute for 15 minutes, then the flow was increased to 8 mL/minute for the remainder of the separation. The product, [F-18]6-HO-BTA N-PrF, eluted at ~20 minutes in a volume of about 16 mL. The fraction containing[F-18]6-HO-BTA-N-PrF was diluted with 50 mL of water and eluted through a Waters C18 SepPak Plus cartridge. The SepPak cartridge was then washed with 10 mL of water, and the product was eluted using 1 mL of ethanol (absol.) into a sterile vial. The solution was diluted with 10 mL of sterile normal saline for intravenous injection into animals. The [F-18]6-HO-BTA-N-PrF product was obtained in 8±4 % (n=8) radiochemical yield at the end of the 120 minute radiosynthesis (not decay corrected) with an average specific activity of 1500 Ci/mmol. The radiochemical and chemical purities of [F-18]6-HO-BTA-N-PrF were assessed by radio-HPLC with UV detection at 350 nm using a Phenomenex Prodigy ODS(3) C18 column (5 µm, 250 x 4.6 mm) eluted with 40% acetonitrile/ 60% 60 mM triethylamine-phosphate buffer (v/v) pH 7.2. [F-18]6-HO-BTA-N-PrF had a retention time of ~12 minutes at a flow rate of 2 mL/minute (k' = 6.1). The radiochemical purity was >99%, and the chemical purity was >90%. The radiochemical identity of [F-18]6-HO-BTA-N-PrF was confirmed by reverse phase radio-HPLC utilizing a quality control sample of the final radiochemical product co-injected with a authentic (cold) standard.

### Ref Example 5: Synthesis of 2-(3'-iodo-4'-aminophenyl)-6-hydroxy benzothiazole

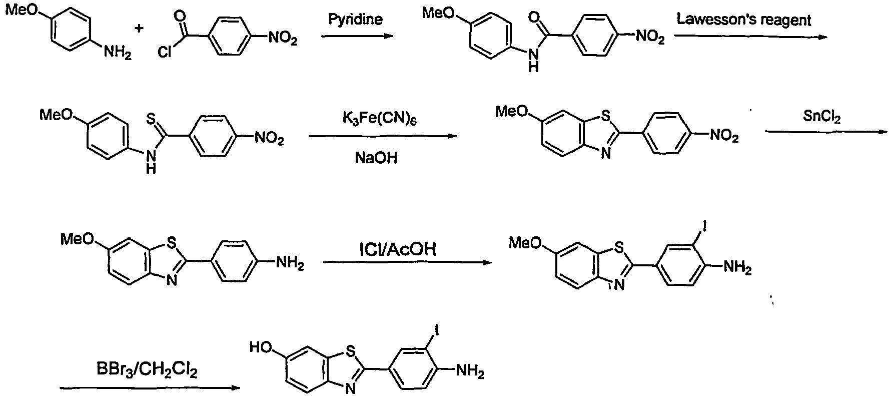

### Preparation of 4-Methoxy-4'-nitrobenzanilide

p-Anisidine (1.0 g, 8.1 mmol) was dissolved in anhydrous pyridine (15 ml), 4-nitrobenzoyl chloride (1.5 g, 8.1 mmol) was added. The reaction mixture was allowed to stand at room temperature for 16 hrs. The reaction mixture was poured into water and the precipitate was collected with filtrate under vacuum pressure and washed with 5% sodium bicarbonate(2 x 10 ml). The product was used in the next step without further purification.
¹HNMR(300MHz, DMSO-d₆) δ: 10.46(s, 1H, NH), 8.37(d, J=5.5Hz, 2H, H-3',5'), 8.17(d, J=6.3Hz, 2H, H-2',6'), 7.48(d, J=6.6Hz, 2H), 6.97(d, J=6.5Hz, 2H), 3.75(s, 3H, MeO).

### Preparation of 4-Methoxy-4'-nitrothiobenzanilide

A mixture of 4-methoxy-4'-nitrothiobenzaniline (1.0 g, 3.7 mmol) and Lawesson's reagent (0.89 g, 2.2 mmol, 0.6 equiv.) in chlorobenzene(15 mL) was heated to reflux for 4 hrs. The solvent was evaporated and the residue was purified with flush column (hexane : ethyl acetate= 4:1) to give 820 mg (77.4%) of the product as orange color solid.
¹HNMR(300MHz, DMSO-d₆) δ: 8.29(d, 2H, H-3',5'), 8.00(d, J=8.5Hz, 2H, H-2',6'), 7.76(d, 2H), 7.03(d, J=8.4Hz, 2H), 3.808.37(d, J=5.5Hz, 2H, H-3',5'), 8.17(d, J=6.3Hz, 2H, H-2',6'), 7.48(d, J=6.6Hz, 2H), 6.97(d, J=6.5Hz, 2H), 3.75(s, 3H, MeO). (s, 3H, MeO).

### Preparation of 6-Methoxy-2-(4-nitrophenyl)benzothiazole

4-Methoxy-4'-nitrothiobenzanilides (0.5 g, 1.74 mmol) was wetted with a little ethanol(~0.5 mL), and 30% aqueous sodium hydroxide solution (556 mg 13.9 mmol. 8 equiv.) was added. The mixture was diluted with water to provide a final solution/suspension of 10% aqueous sodium hydroxide. Aliquots of this mixture were added at 1 min intervals to a stirred solution of potassium ferricyanide (2.29 g, 6.9 mmol, 4 equiv.) in water (5 mL) at 80-90 °C. The reaction mixture was heated for a further 0.5 h and then allowed to cool. The participate was collected by filtration under vacuum pressure and washed with water, purified with flush column (hexane:ethyl acetate= 4:1) to give 130 mg (26%) of the product.
¹HNMR(300MHz, Acetone-d₆) δ: 8.45(m, 4H), 8.07(d, J=8.5Hz, 1H, H-4), 7.69(s, 1H, H-7), 7.22(d, J=9.0Hz, 1H, H-5), 3.90(s, 3H, MeO)

### Preparation of 6-Methozy-2-(4-aminophenyl)benzothiazole

A mixture of the 6-methoxy- 2-(4-nitropheyl)benzothiazoles (22 mg, 0.077 mmol) and tin(II) chloride □omograph(132 mg, 0.45 mmol) in boiling ethanol was stirred under nitrogen for 4 hrs. Ethanol was evaporated and the residue was dissolved in ethyl acetate (10 mL), washed with 1 N sodium hydroxide(2 mL) and water( 5 mL), and dried over MgSO₄. Evaporation of the solvent gave 19 mg (97%) of the product as yellow solid.

### Preparation of 2-(3'-Iodo-4'-aminophenyl)-6-methoxybenzothiazole

To a solution of 2-(4'-aminophenyl)-6-methoxy benzothiazole (22 mg, 0.09 mmol) in glacial acetic acid (2.0 mL) was injected 1 M iodochloride solution in CH₂Cl₂ (0.10 mL, 0.10 mmol, 1.2eq.) under N₂ atmosphere. The reaction mixture was stirred at room temperature for 16 hr. The glacial acetic acid was removed under reduced pressure and the residue was dissolved in CH₂Cl₂. After neutralizing the solution with NaHCO₃, the aqueous layer was separated and extracted with CH₂Cl₂. The organic layers were combined and dried over MgSO₄. Following the evaporation of the solvent, the residue was purified by preparative TLC(Hexanes : ethyl acetate=6:1) to give 2-(4'-amino-3'-iodophenyl)-6-methoxy benzothiazole (25 mg, 76%) as brown solid. ¹HNMR (300MHz, CDCl₃) δ (ppm): 8.35 (d, J=2.0 Hz, 1H), 7.87 (dd, J₁=2.0 Hz, J₂=9.0 Hz, 1H), 7.31 (d, J=2.2 Hz, 1H), 7.04 (dd, J₁=2.2 Hz, J₂=9.0 Hz, 1H), 6.76 (d, J=9.0 Hz, 1H), 3.87 (s, 3H).

### Preparation of 2-(3'-Iodo-4'-aminophenyl)-6-hydroxybenzothiazole

To a solution of 2-(4'-Amino-3'-iodophenyl)-6-methoxy benzothiazole (5) (8.0 mg, 0.02 mmol) in CH₂Cl₂ (2.0 mL) was injected 1 M BBr₃ solution in CH₂Cl₂ (0.20 ml, 0.20 mmol) under N₂ atmosphere. The reaction mixture was stirred at room temperature for 18 hrs. After the reaction was quenched with water, the mixture was neutralized with NaHCO₃. The aqueous layer was extracted with ethyl acetate(3 x 3 mL). The organic layers were combined and dried over MgSO₄. The solvent was then evaporated under reduced pressure and the residue was purified by preparative TLC (Hexanes : ethyl acetate=7:3) to give 2-(3'-iodo-4'-aminophenyl)-6-hydroxybenzothiazole (4.5 mg, 58%) as a brown solid. ¹HNMR (300 MHz, acetone-d₆) δ (ppm): 8.69 (s, 1H), 8.34 (d, J=2.0 Hz, 1H), 7.77 (dd, J₁=2.0 Hz, J₂=8.4 Hz, 1H), 7.76 (d, J=8.8 Hz, 1H), 7.40 (d, J=2.4 Hz, 1H), 7.02 (dd, J₁=2.5 Hz, J₂=8.8 Hz, 1H), 6.94 (d, J=8.5 Hz, 1H), 5.47 (br., 2H). HRMS *m*/*z* 367.9483 (M⁺ calcd for C₁₃H₉N₂OSI 367.9480).

### Ref. Example 6: Synthesis of 2-(3'-iodo-4'-methylaminophenyl)-6-hydroxybenzothiazole

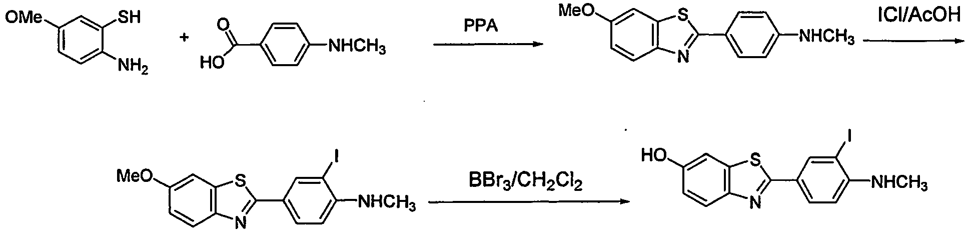

### Preparation of 6-Methoxy-2-(4-methylaminophenyl) benzothiazole

A mixture of 4-methylaminobenzoic acid (11.5 g, 76.2 mmol) and 5-methoxy-2-aminothiophenol (12.5, g, 80 mmol) was heated in PPA (~30 g) to 170°C under N₂ atmosphere for 1.5 hr. The reaction mixture was then cooled to room temperature and poured into 10% K₂CO₃ solution. The precipitate was filtered under reduced pressure. The crude product was re-crystallized twice from acetone/water and THF/water followed by the treatment with active with carbon to give 4.6 g (21%) of 6-Methoxy-2-(4-methylaminophenyl) benzothiazole as a yellow solid. **¹HNMR** (300 MHz, acetone-d₆) δ: 7.84(d, J=8.7Hz, 2H, H-2' 6'), 7.78(dd, J₁=8.8Hz, J₂=1.3Hz, 1H, H-4), 7.52(d, J=2.4Hz, 1H, H-7), 7.05(dd, J₁=8.8Hz, J₂=2.4Hz, H-5), 6.70(d, J=7.6Hz, 2H, H-3' 5'), 5.62(s, 1H, NH), 3.88(s, 3H, OCH₃), 2.85(d, J=6.2Hz, 3H, NCH₃)

### Preparation of 2-(3'-Iodo-4'-methylaminophenyl)-6-methoxy benzothiazole

To a solution of 2-(4'-Methylaminophenyl)-6-methoxy benzothiazole (20 mg, 0.074 mmol) dissolved in glacial acetic acid (2mL) was added Icl (90 µL, 0.15 mmol, 1.2 eq, 1M in CH₂Cl₂) under N₂. The reaction was allowed to stir at room temperature for 18 hr. The glacial acetic acid was then removed under reduced pressure. The residue was dissolved in CH₂Cl₂ and neutralized with NaHCO₃. The aqueous layer was extracted with CH₂Cl₂ and the organic layers were combined, dried over MgSO₄ and evaporated. The residue was purified with preparative TLC (Hexane : EA=2:1) to give 2-(4'-methylamino-3'-iodophenyl)-6-methoxy benzothiazole (8 mg, 27%) as brown solid. ¹HNMR(300MHz, CDCl₃)δ(ppm):8.39(d, J=2.0Hz, 1H), 7.88 (d, J=9.0Hz, 1H), 7.33 (d, J=2.2Hz, 1H), 7.06 (dd, J₁=2.2Hz, J₂=9.0Hz, 1H), 6.58 (d, J=9.0Hz, 1H), 3.89(s, 3H, OCH₃).

### Preparation of 2-(3'-Iodo-4'-methylamino-phenyl)-6-hydroxy benzothiazole

To a solution of 2-(4'-methylamino-3'-iodophenyl)-6-methoxy benzothiazole (12 mg, 0.03 mmol) dissolved in CH₂Cl₂(4mL) was added BBr₃ (400 µl,0.4 mmol, 1M in CH₂Cl₂) under N₂. The reaction was allowed to stir at room temperature for 18 hr. Water was then added to quench the reaction and the solution was neutralized with NaHCO₃, extracted with ethyl acetate (3 x 5 mL). The organic layers were combined, dried over MgSO₄ and evaporated. The residue was purified with preparative TLC (Hexane : EA=7:3) to give 2-(4'-methylamino-3'-iodophenyl)-6-hydroxy benzothiazole (5 mg, 43%) as brown solid. ¹H-NMR(300MHz, CDCl₃) δ(ppm): 8.37 (d, H=2.0Hz, 1H), 7.88 (dd, J₁=2.0Hz, J₂=8.4Hz, 1H), 7.83 (d, J=8.8Hz, 1H), 7.28 (d, J=2.4Hz, 1H), 6.96 (dd, J₁=2.5Hz, J₂=8.8Hz, 1H), 6.58 (d, J=8.5Hz, 1H), 2.96 (s, 3H, CH₃).

### Ref. Example 7: Radiosynthesis of [¹²⁵I]6-OH-BTA-0-3'-1

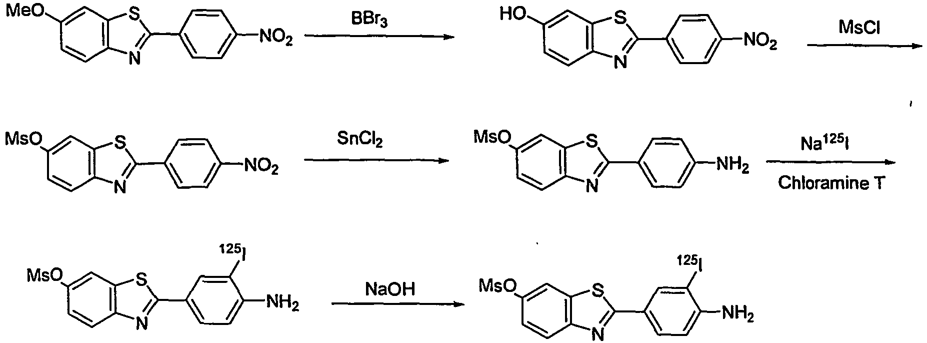

### Preparation of 2-(4'-Nitrophenyl)-6-hydroxybenzothiazole

To a suspension of 2-(4'-nitrophenyl)-6-methoxy benzothiazole (400 mg, 1.5 mmol) in CH₂Cl₂ (10 mL) was added BBr₃ (1M in CH₂Cl₂, 10 mL, 10 mmol). The reaction mixture was stirred at room temperature for 24 hr. The reaction was then quenched with water, and extracted with ethyl acetate (3 x 20 mL). The organic layers were combined and washed with water, dried over MgSO₄, and evaporated. The residue was purified by flash chromatography (silica gel, hexanes : ethyl acetate = 1:1) to give the product as a yellow solid (210 mg, 55%). ¹HNMR(300MHz, Acetone-d₆) δ (ppm): 9.02(s, OH), 8.41(d, J=9.1Hz, 1H),8.33(d, J=9.1Hz, 1H), 7.96(d, J=8.6Hz, 1H), 7.53(d, J=2.4Hz, 1H), 7.15(dd, J1=8.6Hz, J2=2.4Hz, 1H).

### Preparation of 2-(4'- Nitrophenyl)-6-methylsulfoxy benzothiazole

To a solution of 2-(4'-nitrophenyl)-6-hydroxy benzothiazole (50mg, 0.18mmol) dissolved in acetone (7 mL, anhydrous) was added K₂CO₃ (100 mg, 0.72 mmol, powdered) and MsCl (200ul). After stirring for 2 hrs, the reaction mixture was filtered. The filtrate was concentrated and the residue was purified by flash column (silica gel, hexane: ethyl acetate = 4:1) to give 2-(4 -nitrophenyl)-6-methylsulfoxy benzothiazole (44 mg, 68%) as pale yellow solid. ¹HNMR(300MHz, acetone-d₆) δ (ppm): 8.50-8.40(m, 4H), 8.29(d, J=2.3Hz, 1H), 8.23(d, J=8.9Hz, 1H), 7.61(dd, J₁=2.3Hz, J₂=8.9Hz, 1H).

### Preparation of 2-(4'- Aminophenyl)-6-methylsulfoxy benzothiazole

To a solution of 2-(4'-nitrophenyl)-6-methylsulfoxy benzothiazole (35mg, 0.10mmol) dissolved in ethanol (10 mL) was added SnCl₂.2H₂O (50mg). The reaction mixture was heated to reflux for 1.5 hr. The solvent was then removed under reduced pressure. The residue was dissolved in ethyl acetate (10 mL), washed with 1N NaOH, water, dried over MgSO₄. Evaporation of the solvent afforded 2-(4'- aminophenyl)-6-methylsulfoxy benzothiazole (21mg, 65%) as pale brown solid. ¹HNMR(300MHz, CDCl₃) δ (ppm): 8.02(d, J=6.2Hz, 1H), 7.92(d, J=8.7Hz, 2H), 7.84(d, J=2.4Hz, 1H), 7.38(dd, J₁=2.4Hz, J₂=6.2Hz, 1H), 6.78(d, J=8.7Hz, 2H), 2.21(s, 3H, CH₃).

### Ref. Example 8: Radiosynthesis of [¹²⁵I]6-OH-BTA-1-3'-I

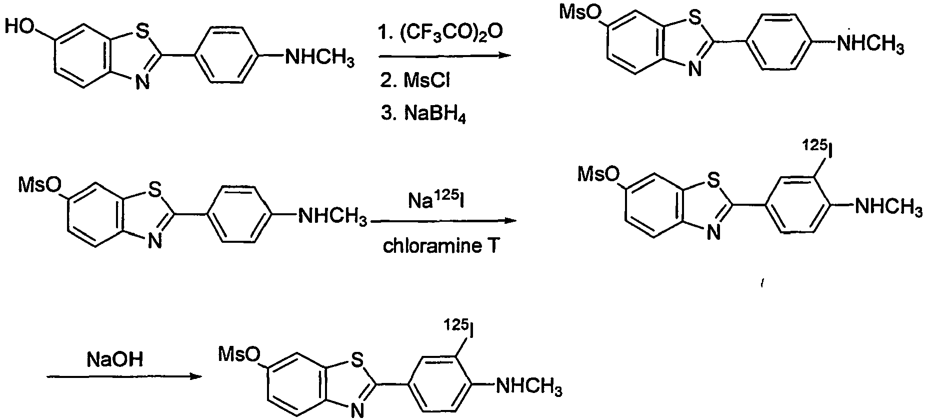

To a solution of 2-(4'-methylaminophenyl)-6-hydroxy benzothiazole (300 mg, 1.17 mmol) dissolved in CH₂Cl₂ (20mmL) was added Et₃N (2 mL) and trifluoroacetic acid (1.5 mL). The reaction mixture was stirred at room temperature for 3 h. The solvent was removed under reduced pressure and the residue was dissolved in ethyl acetate (30 mL), washed with NaHCO₃ solution. Brine, water, and dried over MgSO₄. After evaporation of the solvent, the residue was dissolved in acetone (20 ml, pre-dried over K₂CO₃), K₂CO₃ (1.0 g, powered) was added followed by MsCl (400 mg, 3.49 mmol). The reaction mixture was stirred at room temperature and monitored with TLC □omog starting material disappeared. The residue was then filtrated. The filtrate was evaporated under reduced pressure. The residue was dissolved in ethyl acetate (30 mL), washed with NaHCO₃ solution. Brine, water, and dried over MgSO₄. After evaporation of the solvent, the residue was dissolved in EtOH and NaBH₄ was added. The reaction mixture was stirred at room temperature for 2 h. The solvent was evaporated and the residue was dissolved in water, extracted with ethyl acetate (20 ml x 3), the extracts were combined and dried over MgSO₄. After evaporation of the solvent, the residue was purified with flash column(hexanes/ethyl acetate = 8:1) to give the product (184mg, 47.0%) as brown solid. ¹HNMR(300MHz, CDCl₃) δ (ppm): 7.94(d, J=8.8Hz, 1H), 7.87(d, J=8.7Hz, 2H), 7.77(d, J=2.3Hz, 1H), 7.30(dd, J₁=8.8Hz, J₂=2.3Hz, 1H), 6.63(d, J=8.7Hz, 2H), 3.16(s, CH₃), 2.89(s, NCH₃).

### General procedures for radiolabelling:

To a solution of 2-(4'-aminophenyl)-6-methanesulfonoxy benzothiazole or 2-(4'-methylaminophenyl)-6-methylsulfoxy benzothiazole (1 mg) in 250 µL acetic acid in a sealed vial was added 40 µL of chloramines T solution (28 mg dissolved in 500 µL acetic acid) followed by 27 µL (ca. 5 mCi) of sodium [¹²⁵I]iodide (specific activity 2,175 Ci/mmol). The reaction mixture was stirred at r.t. for 2.5 hrs and quenched with saturated sodium hydrogensulfite solution. After dilution with 20 ml of water, the reaction mixture was loaded onto C8 Plus SepPak and eluted with 2 ml methanol. For deprotection of the methanesulfonyl group, 0.5 ml of 1 M NaOH was added to the eluted solution of radioiodinated intermediate. The mixture was heated at 50 °C for 2 hours. After being quenched by 500 µL of 1 M acetic acid, the reaction mixture was diluted with 40 mL of water and loaded onto a C8 Plus SepPak. The radioiodinated product, having a radioactivity of ca. 3 mCi, was eluted off the SepPak with 2 mL of methanol. The solution was condensed by a nitrogen stream to 300 µL and the crude product was purified by HPLC on a Phenomenex ODS column (MeCN/TEA buffer, 35:65, pH 7.5, flow rate 0.5 mL/min up to 4 min, 1.0 mL/min at 4-6 min, and 2.0 mL/min after 6 min, retention time 23.6). The collected fractions were loaded onto a C8 Plus SepPak. Elution with 1 mL of ethanol gave ca. 1 mCi of the final radioiodinated product.

### Example 9: Synthesis of 2-(4'-aminophenyl)-benzothiazole derivatives

Route 1: Example of the synthesis of 6-MeO-BTA-0, -1, -2, which are representative of the group of thioflavin compounds (Shi et al., "Antitumor Benzothiazoles. 3. Synthesis of 2-(4-Aminophenyl)benzothiazoles and Evaluation of Their Activities against Breast Cancer Cell Lines in Vitro and in Vivo" J Med. Chem. 39:3375-3384, 1996).

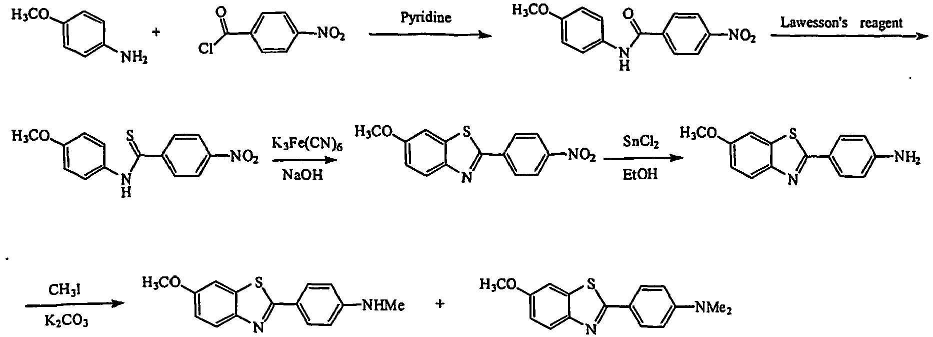

### Preparation of 4-Methoxy-4'-nitrobenzanilide

p-Anisidine (1.0 g, 8.1 mmol) was dissolved in anhydrous pyridine (15 ml), 4-nitrobenzoyl chloride (1.5 g, 8.1 mmol) was added. The reaction mixture was allowed to stand at room temperature for 16 hrs. The reaction mixture was poured into water and the precipitate was collected with filtrate under vacuum pressure and washed with 5% sodium bicarbonate(2 x 10 ml). The product was used in the next step without further purification. ¹HNMR(300MHz, DMSO-d₆) δ: 10.46(s, 1H, NH), 8.37(d, J=5.5Hz, 2H, H-3',5'), 8.17(d, J=6.3Hz, 2H, H-2',6'), 7.48(d, J=6.6Hz, 2H), 6.97(d, J=6.5Hz, 2H), 3.75(s, 3H, MeO).

### Preparation of 4-Methoxy-4'-nitrothiobenzanilide

A mixture of 4-methoxy-4'-nitrothiobenzaniline (1.0 g, 3.7 mmol) and Lawesson's reagent (0.89 g, 2.2 mmol, 0.6 equiv.) in chlorobenzene(15 mL) was heated to reflux for 4 hrs. The solvent was evaporated and the residue was purified with flush column (hexane : ethyl acetate= 4:1) to give 820 mg (77.4%) of the product as orange color solid. ¹HNMR(300MHz, DMSO-d₆) δ: 8.29(d, 2H, H-3',5'), 8.00(d, J=8.5Hz, 2H, H-2',6'), 7.76(d, 2H), 7.03(d, J=8.4Hz, 2H), 3.808.37(d, J=5.5Hz, 2H, H-3',5'), 8.17(d, J=6.3Hz, 2H, H-2',6'), 7.48(d, J=6.6Hz, 2H), 6.97(d, J=6.5Hz, 2H), 3.75(s, 3H, MeO). (s, 3H, MeO).

### Preparation of 6-Methoxy-2-(4-nitrophenyl)benzothiazole

4-Methoxy-4'-nitrothiobenzanilides (0.5 g, 1.74 mmol) was wetted with a little ethanol(∼0.5 mL), and 30% aqueous sodium hydroxide solution (556 mg 13.9 mmol. 8 equiv.) was added. The mixture was diluted with water to provide a final solution/suspension of 10% aqueous sodium hydroxide. Aliquots of this mixture were added at 1 min intervals to a stirred solution of potassium ferricyanide (2.29 g, 6.9 mmol, 4 equiv.) in water (5 mL) at 80-90 °C. The reaction mixture was heated for a further 0.5 h and then allowed to cool. The participate was collected by filtration under vacuum pressure and washed with water, purified with flush column (hexane:ethyl acetate= 4:1) to give 130 mg (26%) of the product. ¹HNMR(300MHz, Acetone-d₆) δ: 8.45(m, 4H), 8.07(d, J=8.5Hz, 1H, H-4), 7.69(s, 1H, H-7), 7.22(d, J=9.0Hz,1H, H-5), 3.90(s, 3H, MeO)

### Preparation of 6-Methoxy-2-(4-aminophenyl)benzothiazole

A mixture of the 6-methoxy- 2-(4-nitropheyl)benzothiazoles (22 mg, 0.077 mmol) and tin(II) chloride □omograph(32 mg, 0.45 mmol) in boiling ethanol was stirred under nitrogen for 4 hrs. Ethanol was evaporated and the residue was dissolved in ethyl acetate (10 mL), washed with 1 N sodium hydroxide(2 mL) and water( 5 mL), and dried over MgSO₄. Evaporation of the solvent gave 19 mg (97%) of the product as yellow solid.

### Preparation of 6-Methoxy-2-(4-methylaminophenyl)benzothiazole and 6-Methoxy-2-(4-dimethylaminophenyl)benzothiazole

A mixture of 6-methoxy-2-(4-aminophenyl)benzothiazole (15 mg, 0.059 mmol), MeI (8.3 mg, 0.060 mmol) and K₂CO₃ (100 mg, 0.72 mmol) in DMSO(anhydrous, 0.5 ml) was heated at 100°C for 16 hrs. The reaction mixture was purified by reverse phase TLC (MeOH:H₂O=7:1) to give 2.0 mg (13.3%) of 6-methoxy-2-4-methylaminophenylbenzothiazole and 6 mg (40%) of 6-methoxy-2-(4-dimethylaminophenyl)benzothiazole. ¹HNMR of 6-methoxy-2-4-methylaminophenylbenzothiazole (300MHz, Acetone-d₆) δ: 7.85(d, J=8.7Hz, 2H, H-2' 6'), 7.75(dd, J=8.8Hz, J=1.3Hz, 1H, H-4), 7.49(d, J=2.4Hz, 1H, H-7), 7.01(dd, J=8.8Hz, J=2.4Hz, H-5), 6.78(d, J=7.6Hz, 2H, H-3' 5'), 3.84(s, 3H, MeO), 2.91(s, 3H, Nme), ¹HNMR of 6-methoxy-2-(4-dimethylaminophenyl)benzothiazole (300MHz, Acetone-d₆)δ: 7.85(d, J=8.7Hz, 2H, H-2' 6'), 7.75(dd, J=8.8Hz, J=1.3Hz, 1H, H-4), 7.49(d, J=2.4Hz, 1H, H-7), 7.0 1 (dd, J=8.8Hz, J=2.4Hz, H-5), 6.78(d, J=7.6Hz, 2H, H-3' 5'), 3.84(s, 3H, MeO), 3.01 (s, 6H,Nme₂),

Following the same strategy as above, the other 2-(4'-aminophenyl)-benzothiazole derivatives may be synthesized by substituting the appropriate substituted aniline derivative (e.g. 2-, 3-, or 4-methylaniline) and the appropriate 4-nitro-benzoyl chloride derivative (e.g. 2- or 3-methyl-4-nitro-benzoyl chloride).

### Example 10: Synthesis of BTA Derivatives without substitution

Route 2: Example of the synthesis of BTA-0, -1, -2 compounds, which are representative of the group of thioflavin compounds (Garmaise et al., "Anthelmintic Quaternary Salts. III. Benzothiazolium Salts" J. Med. Chem. 12:30-36 1969) (reference numbers of the names compounds below refer to the synthetic scheme shown):

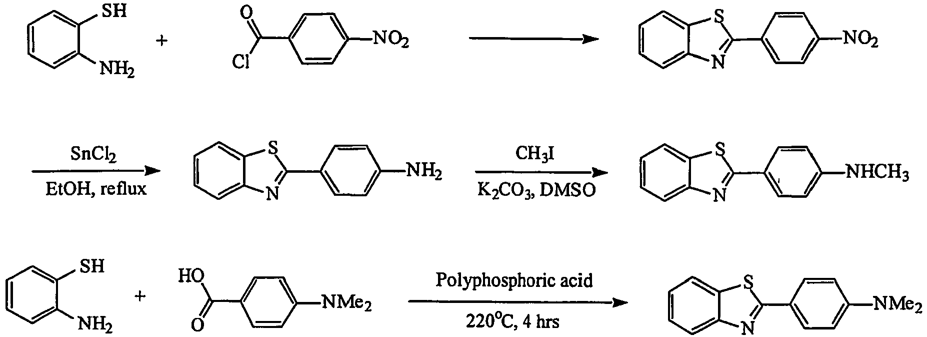

### Preparation of 2-(4-Nitrophenyl)benzothiazole

A solution of 4-nitrobenzoyl chloride (1.49 g, 8.0 mmol) in benzene (anhydrous, 10 mL) was added dropwise to 2-aminothiophenol (1.0 g, 8.0 mmol in 10 ml of benzene) at room temperature. The reaction mixture was allowed to stir for 16 hr. The reaction was quenched with water (20 mL). The aqueous layer was separated and extracted with ethyl acetate (3 x 10 ml). The combined organic layers were dried and evaporated. The crude product was purified with flush column, (hexane: ethyl acetate= 85:15) to give 1.5 g (73.2%) of product as light yellow solid.

### Preparation of 2-(4-Aminophenyl)benzothiazole

A mixture of 2-(4-nitrophenyl)benzothiazole (105 mg, 0.40 mmol) and tin(II) chloride □omograph (205 mg, 0.91mmol) in ethanol (20 mL) was refluxed under N₂ for 4 hrs. After removing ethanol by vacuum evaporation. The residue was dissolved into ethyl acetate (20 ml), and washed with NaOH solution (1N, 3 x 20 ml) and water (3 x 20ml), dried and evaporated to dryness to give 102 mg (97%) of the product

### Preparation of 2-(4-Methylaminophenyl)benzothiazole and 2-(4-dimethylaminophenyl)benzothiazole

A mixture of 2-(4-aminophenyl)benzothiazole (15mg, 0.066mmol), MeI (9.4 mg, 0.066 mg) and K₂CO₃ (135 mg, 0.81mmol) in DMSO (anhydrous, 0.5 ml) was heated at 100°C for 16 hrs. The reaction mixture was purified by reverse phase TLC (MeOH:H₂O=6:1) to give 1.5 mg (10%) of 2-(4-methylminophenyl)benzothiazole and 2.5 mg (16.7%) of 2-(4-dimethylaminophenyl)benzothiazole.

### Preparation of 2-(4-Dimethylaminophenyl)benzothiazole

The mixture of 2-aminothiophenol (0.5 g, 4.0 mmol) 4-dimethylaminobenzoic acid (0.66 g, 4.0 mmol) and PPA (10 g) was heated to 220°C for 4 hrs. The reaction mixture was cooled to room temperature and poured into a solution of 10% potassium carbonate (∼400mL). The residue was collected by filtration under vacuum pressure to give 964 mg of the product, which was ca. 90% pure based on the ¹HNMR analysis. Recrystalization of 100 mg in MeOH gave 80 mg of the pure product. ¹HNMR(300MHz, Acetone-d₆) δ: 7.12(d, J=7.7Hz, 1H, H-7), 7.01(d, J=9.0Hz, 1H, H-4), 6.98(d, J=9.1Hz, 2H, H-2',6'), 6.56(t, J=7.8Hz, J=7.3Hz,, 1H, H-5 or H-6), 5.92(d, J=8.9Hz, 1H, H-3',5'), 2.50(s, 6H, Nme₂).

Following the same strategy as above, the other 2-(4'-aminophenyl)-benzothiazole derivatives may be synthesized by substituting appropriate 4-nitro-benzoyl chloride derivative (e.g. 2- or 3-methyl-4-nitro-benzoyl chloride) or appropriate 4-dimethylaminobenzoic acid derivative (e.g. 2- or 3-methyl-4-dimethylamino-benzoic acid).

### Example 11. Synthesis of iodinated compounds

Route 3: Example of the synthesis of 2-(3'-Iodo-4'-aminophenyl)-6-hydroxybenzathiazole, which is representative for the synthesis of other iodinated compounds (reference numbers of the names compounds below refer to the synthetic scheme shown).

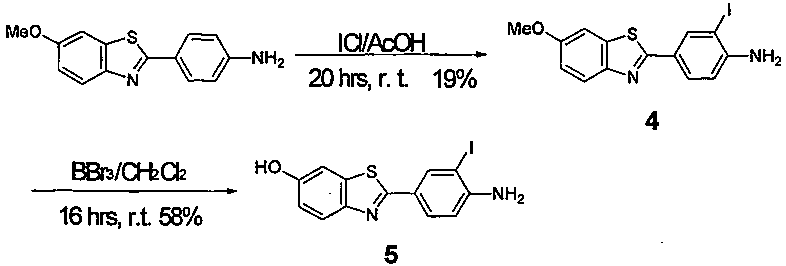

### Preparation of 2-(3'-Iodo-4'-aminophenyl)-6-methoxybenzothiazole

To a solution of 2-(4'-aminophenyl)-6-methoxybenzathiazole (22 mg, 0.09 mmol) in glacial acetic acid (2.0 mL) was injected 1 M iodochloride solution in CH₂Cl₂ (0.10 mL, 0.10 mmol, 1.2eq.) under N₂ atmosphere. The reaction mixture was stirred at room temperature for 16 hr. The glacial acetic acid was removed under reduced pressure and the residue was dissolved in CH₂Cl₂. After neutralizing the solution with NaHCO₃, the aqueous layer was separated and extracted with CH₂Cl₂. The organic layers were combined and dried over MgSO₄. Following the evaporation of the solvent, the residue was purified by preparative TLC(Hexanes : ethyl acetate=6:1) to give 2-(4'-amino-3'-iodophenyl)-6-methoxybenzathiazole (25 mg, 76%) as brown solid. ¹HNMR (300MHz, CDCl₃) δ (ppm): 8.35 (d, J=2.0 Hz, 1H), 7.87 (dd, J₁=2.0 Hz, J₂=9.0 Hz, 1H), 7.31 (d, J=2.2 Hz, 1H), 7.04 (dd, J₁=2.2 Hz, J₂=9.0 Hz, 1H), 6.76 (d, J=9.0 Hz, 1H), 3.87 (s, 3H).

### Preparation of 2-(3'-Iodo-4'-aminophenyl)-6-hydroxybenzathiazole

To a solution of 2-(4'-Amino-3'-iodophenyl)-6-methoxybenzathiazole (8.0 mg, 0.02 mmol) in CH₂Cl₂ (2.0 mL) was injected 1 M BBr₃ solution in CH₂Cl₂ (0.20 ml, 0.20 mmol) under N₂ atmosphere. The reaction mixture was stirred at room temperature for 18 hrs. After the reaction was quenched with water, the mixture was neutralized with NaHCO₃. The aqueous layer was extracted with ethyl acetate(3 x 3 mL). The organic layers were combined and dried over MgSO₄. The solvent was then evaporated under reduced pressure and the residue was purified by preparative TLC (Hexanes : ethyl acetate=7:3) to give 2-(3'-iodo-4'-aminophenyl)-6-hydroxybenzothiazole (4.5 mg, 58%) as a brown solid. ¹HNMR (300 MHz, acetone-d₆) δ (ppm): 8.69 (s, 1H), 8.34 (d, J=2.0 Hz, 1H), 7.77 (dd, J₁=2.0 Hz, J₂=8.4 Hz, 1H), 7.76 (d, J=8.8 Hz, 1H), 7.40 (d, J=2.4 Hz, 1H), 7.02 (dd, J₁=2.5 Hz, J₂=8.8 Hz, 1H), 6.94 (d, J=8.5 Hz, 1H), 5.47 (br., 2H). HRMS *m*/*z* 367.9483 (M⁺ calcd for C₁₃H₉N₂OSI 367.9480).

### Biological Examples

### Example 1: Determination of Affinity for Aβ and brain Uptake of Thioflavin Derivatives

Initial binding studies using synthetic Aβ(1-40) were conducted to determine if the four compounds shown below bound significantly to sites of amyloid deposits in the brain.

| Structures | Kᵢ (nM) | logP | 2 min (%ID/g) | 30 min (%ID/g) | 2:30 min rati |
|---|---|---|---|---|---|
| 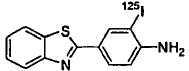 | 8.32 | 3.17 | 9.08 | 3.4 | 2.7 |
| 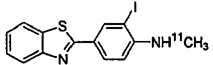 | 4.94 | 3.90 | 4.40 | 2.68 | 1.6 |
| 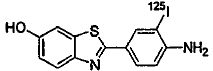 | 11.1 | 1.65 | 5.64 | 0.36 | 15.7 |
| 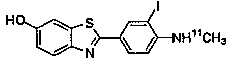 | 3.22 | 2.35 | 7.76 | 2.66 | 2.91 |

The data shown in Table 1 indicates that these compounds displayed relatively high affinity for Aβ, with Ki values <10 nM, and readily entered mouse brain with uptake values >0.4 %ID/g*kg (or>13% ID/g for 30 g animals). Moreover, the 30 min brain radioactivity concentration values were less than 0.1 %ID/g*kg, resulting in 2 min-to-30 min concentration ratios >4.

### Example 2: Staining amyloid deposits in post-mortem AD and Tg mouse brain

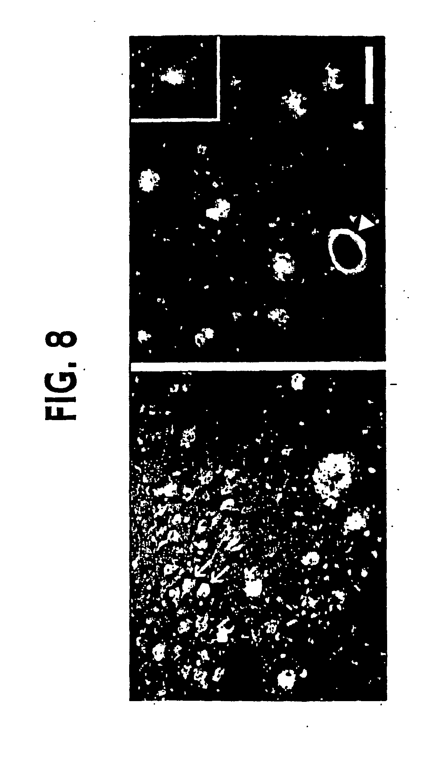

Postmortem brain tissue sections from AD brain and an 8 month old transgenic PS1/APP mouse were stained with unlabeled BTA-1. The PS1/APP mouse model combines two human gene mutations known to cause Alzheimer's disease in a doubly transgenic mouse which deposits Aβ fibrils in amyloid plaques in the brain beginning as early as 3 months of age. Typical fluorescence micrographs are shown in Figure 8, and the staining of amyloid plaques by BTA-1 in both postmortem AD and PS1/APP brain tissue is clearly visible. Cerebrovascular amyloid also was brightly stained (Fig. 8, right). The other characteristic neuropathological hallmark of AD brain, neurofibrillary tangles (NFT), are more faintly stained by BTA-1 in AD brain (Fig. 8, left). NFT have not been observed in transgenic mouse models of amyloid deposition.

### Example 3. In vivo labeling and detection of amyloid deposits in transgenic mice

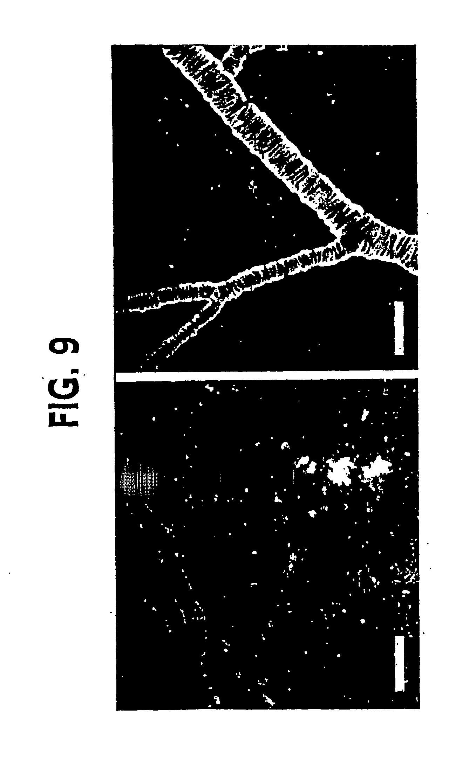

Three 17 month-old PS1/APP transgenic mice were injected intraperitoneally (ip) with a single dose of 10 mg/kg of BTA-1 in a solution of DMSO, propylene glycol, and pH 7.5 PBS (v/v/v 10/45/45). Twenty-four hours later, multiphoton fluorescence microscopy was employed to obtain high resolution images in the brains of living mice using a cranial window technique. Typical in vivo images of BTA-1 in a living PS1/APP mouse are shown in Figure 9, and plaques and cerebrovascular amyloid are clearly distinguishable. The multiphoton microscopy studies demonstrate the in vivo specificity of BTA-1 for Aβ in living PS1/APP transgenic mice.

### Example 4. Specificity of the Inventive Compounds for Alzheimer plaques over Alzheimer tangles

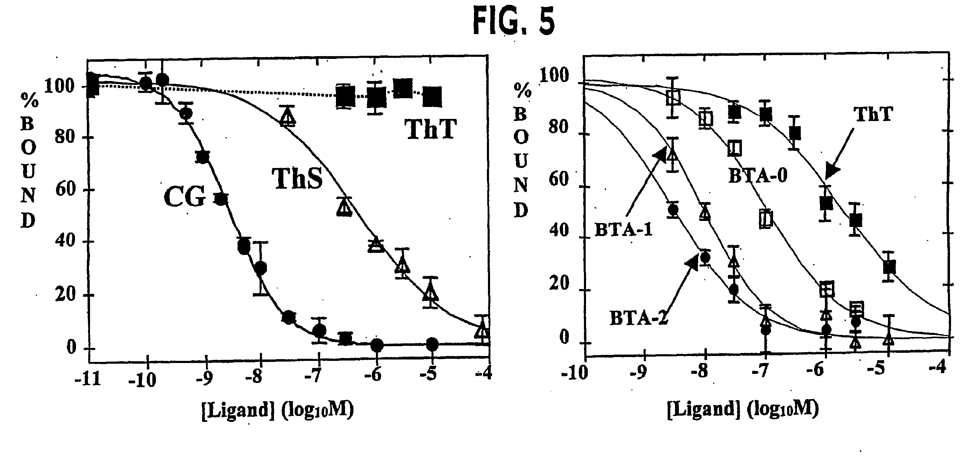

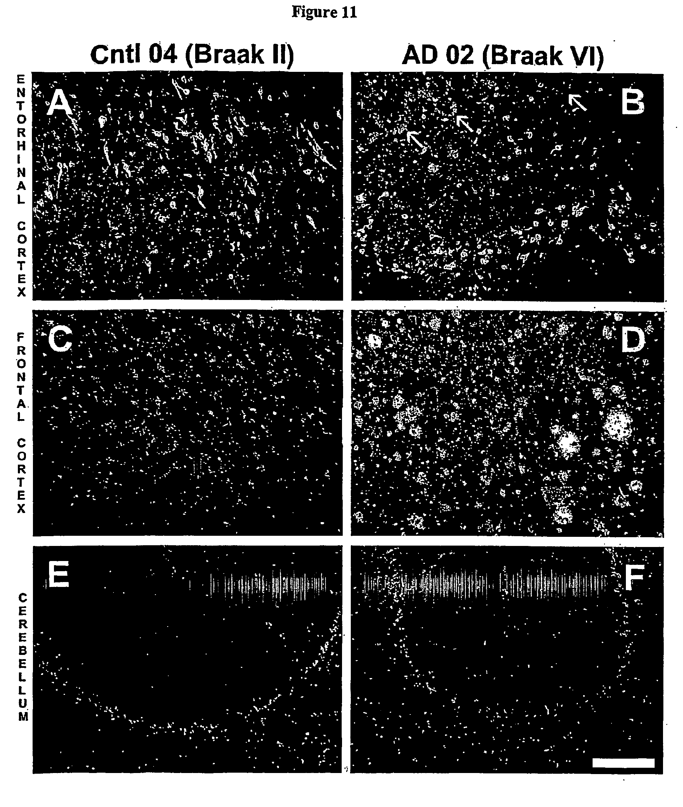

In order to address the relative contributions of [³H]BTA-1 binding to Aβ and tau deposits in the frontal gray of AD brain, [³H]BTA-1 binding was compared in homogenates from entorhinal cortex (EC), frontal gray and cerebellum from a typical AD brain and a Braak stage II control brain. This control brain had frequent numbers of NFT in the entorhinal cortex (Fig. 5A), but no neuritic or diffuse plaques in any area of the brain (Fig. 11C). The NFT numbers in the EC of Cntl 04 were similar to the numbers found in many AD cases (Fig. 11B). [³H]BTA-1 binding in the NFT-rich EC region of this Cntl 04 brain was no greater than [³H]BTA-1 binding in the plaque- and NFT-free cerebellum and frontal gray from this brain (Figure 11, Table). A similar survey of these same brain areas in a Braak VI AD brain (Figure 11, Table), showed low binding in cerebellum and EC and over ten-fold higher levels in frontal gray where there are frequent numbers of neuritic plaques (Fig. 11D). The extensive NFT pathology in the EC of the Cntl and AD brains, coupled with the low [³H]BTA-1 binding in the EC suggests that either the BTA-1 binding to NFT seen at 100 nM concentrations of BTA-1 does not occur at 1.2 nM, or that, at low nanomolar concentrations, the total absolute amount of [³H]BTA-1 binding to NFT deposits is small in comparison to the amount of [³H]BTA-1 bound to Aβ deposits in the plaques and cerebrovascular amyloid of AD frontal gray. The AD brain showed diffuse amyloid plaque deposits in the EC (Fig. 11B) which did not appear to produce significant [³H]BTA-1 binding. The frontal cortex had extensive amyloid plaques which were both compact and diffuse and were associated with high levels of [³H]BTA-1 binding (Fig. 11D and Table).

### Example 5: In vivo mouse brain entry studies

Experiments to assess brain penetration of 2-(3'-¹²⁵I-iodo-4'-amino-phenyl)-benzothiazol-6-ol (Compound A), 2-(3-[¹⁸F]-fluoro-4-methylamino-phenyl)-benzothiazol-6-ol (Compound B), and 2-[4-(3-¹⁸F-fluoro-propylamino)-phenyl]benzothiazol-6-ol (Compound C) were performed in young, wild type mice that had no amyloid deposits in their brain. This study reflects brain entry and clearance from normal brain tissue. A necessary criterion for a good PET imaging agent is rapid clearance from brain areas that do not contain the targeted binding site. A measure of non-specific binding clearance rate is provided by the ratio of the 2 minutes-to-30 minutes (%ID-kg)/g values.

Studies were performed in female Swiss-Webster mice (23-35 g) in accordance with the Guide for the Care and Use of Laboratory Animals adopted by NIH and with the approval of the local Institutional Animal Care and Use Committee. The mice were injected in a lateral tail vein with 0.37-3.7 MBq (10-100 µCi) of a high specific activity (∼2.0/µmol) Compound A, Compound B or Compound C contained in ≤ 0.10 mL of a solution of 95% isotonic saline and 5% ethanol. The mice were anesthetized and killed by cardiac excision following cardiac puncture to obtain arterial blood samples at 2 minutes or 30 minutes post-injection. The mouse brains were rapidly excised and divided into the cerebellum and the remaining whole brain (including brain stem) fractions. The brain samples were counted in a gamma well-counter, and the counts were decay-corrected to the time of injection relative to ¹²⁵I or ¹⁸F standards prepared from the injection solution to determine the percent injected dose (%ID) in the samples. The brain samples were weighed to determine the percent injected dose per gram tissue (%ID/g), and this quantity was multiplied by the whole body weight (in kg) to determine the body-weight normalized radioactivity concentration [(%ID-kg)/g] of each tissue sample. Compound A, Compound B and Compound C displayed relatively high brain entry at early time points and fast clearance at later time points. The radioactivity concentrations (%ID-kg/g) at 2 minutes and 30 minutes and the 2 minutes-to-30 minutes ratios are presented in Table 2 below.

**TABLE 2**

| | Radioactivity Conc. | Radioactivity Conc. | 2 min./30 min. |
|---|---|---|---|
| | At 2 min. (%ID-kg/g) | At 30 min. (%ID-kg/g) | Ratio |
| Compound A | 0.141 | 0.009 | 16 |
| Compound B | 0.29 | 0.030 | 10 |
| Compound C | 0.17 | 0.011 | 16 |

### Example 7: In vivo baboon imaging studies

PET imaging studies in adult baboons (*Papio anubis*) (weight 15-35 kg, ages 6-12 years) were performed with Compound B and Compound C in accordance with the Guide for the Care and Use of Laboratory Animals adopted by NIH and with the approval of the local Institutional Animal Care and Use Committee. Prior to PET imaging, the animals were initially sedated with ketamine (10-15 mg/kg, i.m.), given atropine (0.5 mg, i.m.) to control salivation and heart rate, and intubated. The baboons were subsequently maintained on a ventilator with isofluorane (0.5-1.25%) anesthesia and medical air. Pancuronium bromide was administered as necessary (intravenously, up to 0.06 mg/kg/hour, titrated to effect) to keep the animals immobilized during the study. A femoral artery catheter was inserted to monitor blood pressure and sample arterial blood, and an intravenous catheter was placed in an antecubital vein for radiotracer injection and to administer fluids as necessary throughout the course of the imaging study. Blood pressure, heart and respiratory rates, and expired CO₂ and oxygen saturation levels were monitored continuously during the PET studies. The baseline rectal body temperature (∼37°C) was maintained using a heating blanket (Gaymar, Orchard Park, NY) and temperature regulator (Yellow Springs Instruments, Yellow Springs, OH). Prior to scanning, the baboon's head was fixed so that the image planes were acquired approximately parallel to the orbital-meatal line.

PET data were acquired using an ECAT HR+ PET scanner (CTI PET Systems, Knoxville, TN) in 3D imaging mode (63 parallel slices; 15.2 cm axial field-of-view; 4.1 mm full-width half-maximum in-plane resolution). A Neuro-Insert (CTI PET Systems) was used to reduce the contribution of scattered photon events. After the baboons were positioned in the PET scanner, a windowed transmission scan (10-15 minutes) was obtained for attenuation correction of the PET emission data using rotating ⁶⁸Ge/⁶⁸Ga rods. Compound B and Compound C were administered intravenously over 20 seconds, and a dynamic series of PET scans were acquired over 90 minutes using 26 frames of increasing length (6 x 20 seconds; 4 x 30 seconds; 6 x 60 seconds; 4 x 5 minutes; 6 x 10 minutes). Approximately 185 MBq (∼5 mCi) of a high specific activity (>14.8 GBq/µmol) Compound B or Compound C was injected in a baboon. In other studies, 148-296 MBq (4-8 mCi) of a high specific activity (>18.5 GBq/µmol) reference PET radiotracer was injected, including either [¹¹C](+)-McN5652, [carbonyl-¹¹C]WAY100635, or [¹⁸F]altanserin. The PET data were reconstructed using a Hanning filter (Nyquist cut-off) and corrected for decay, photon attenuation, and scatter.

An MRI scan was obtained for each baboon using a 1.5T GE Signa scanner (GE Medical Systems, Milwaukee, WI) equipped with a standard head coil. A volumetric spoiled gradient recalled (SPGR) MR sequence with parameters for high contrast among gray matter, white matter, and cerebral spinal fluid (CSF) was acquired in the coronal plane (TE = 5, TR = 24, flip angle = 40°, slice thickness =1.5 mm, NEX = 2, field of view 12 cm, voxel size = 0.94 x 1.25 x 1.5 mm). Each individual baboon's MR image was coregistered to the PET data using the automated image registration (AIR) algorithm for cross-modality image alignment and reslicing. The initial 16 frames (0-9 minutes post-injection) of the dynamic PET images were summed together into images consisting of a single frame. Prior to co-registration, both the MR and summed PET images were edited using the ANALYZE software package (Mayo Clinic, Rochester, MN) to remove extracerebral tissues that could possibly confound the co-registration process. The edited MR images were then coregistered to the summed PET image and resliced to yield MR images in the same spatial orientation and resolution as the summed PET images. The co-registration of MR and PET datasets in the baboon has been demonstrated to be a reliable and robust application of the AIR method.

Regions of interest (ROIs) were defined on the coregistered MR image and applied to the dynamic PET datasets to determine regional time-activity data for white matter (cerebral white matter posterior to prefrontal cortex and anterior to lateral ventricles), temporal cortex, cerebellum (cerebellar cortex), and other brain areas (data not shown). The PET time-activity data were converted to units of microcuries per milliliter using a phantom-based calibration factor and were subsequently normalized to the injected dose and body mass of the animal ((%ID-kg)/g).

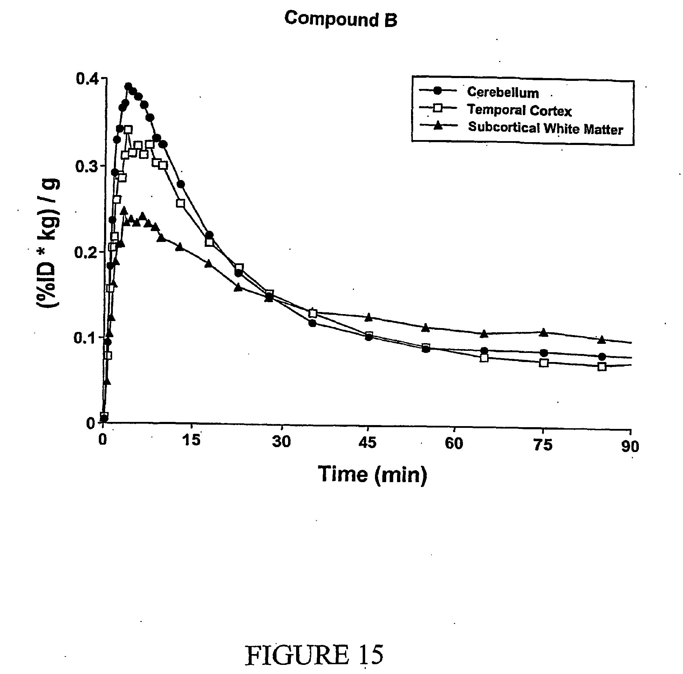

Figure 15 shows a representative PET time-activity curve (TAC) of radioactivity in three brain regions of a baboon following the intravenous injection of Compound B. The TACs indicate excellent brain penetration of radioactivity at early time points (about 0.40 % ID-kg/g, in reasonable agreement to the brain penetration of Compound B in mice at 2 minutes post-injection) in all three regions and relatively rapid clearance of the regional radioactivity from 0-90 minutes post-injection in the brain of this control baboon. Regions of brain containing higher levels of white matter demonstrated somewhat higher (∼30%) concentrations of radioactivity at 90 minutes than regions that were dominated by gray matter such as temporal cortex. The concentration of radioactivity in baboon cortex was nearly identical to that in the cerebellar cortex at all time points. The rate of clearance of radioactivity was considerably slower from baboon brain than from mouse brain, with Compound B exhibiting a clearance half-time of about 17 minutes from baboon brain gray matter. The radiotracer Compound B exhibited an early-to-late brain radioactivity concentration in baboon brain of about 4 indicating that only about 25% of the peak maximum radioactivity remained in brain at later time points. These results were consistent with the expected absence of amyloid plaques in the brains of these control animals and indicated that very little radioactivity was retained in normal baboon brain. Comparison of the *in vivo* behavior of Compound B in baboon brain to that of the entry and clearance of other successful PET radioligands in a reference brain region devoid of specific binding sites (i.e., cerebellum) was useful.

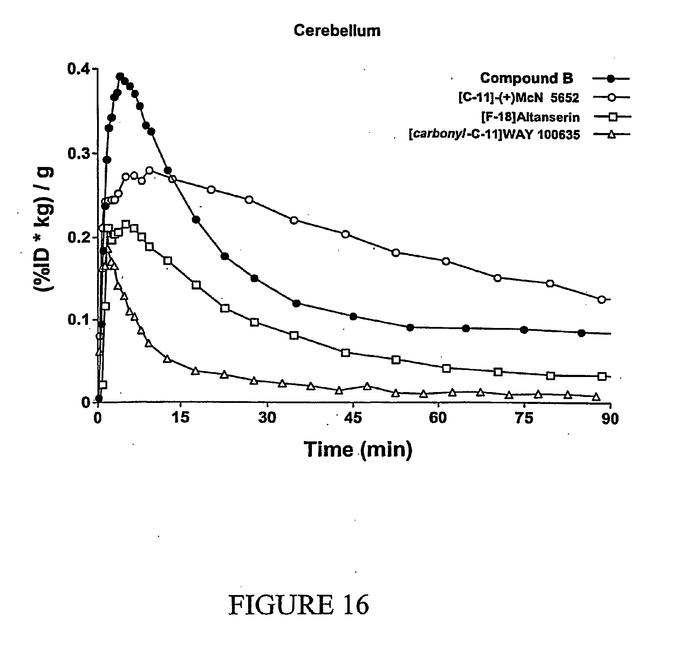

Figure 16 compares the cerebellar TACs in baboons of [carbonyl-¹¹C]WAY100635, [¹¹C](+)-McN5652, [¹⁸F]altanserin and Compound B. The relatively rapid non-specific binding clearance rates of [carbonyl-¹¹C]WAY100635 and [¹⁸F]altanserin are important to the success of these PET radioligands for imaging the serotonin 5-HT_{1A} and serotonin 5-HT_{2A} receptor systems. In contrast, the relatively slow *in vivo* clearance of [¹¹C](+)-McN5652 has limited the usefulness of this radioligand for imaging the serotonin transporter system. The brain clearance properties of Compound B indicated that the relatively rapid rate of non-specific clearance of this radiotracer (t_{1/2} = 17 minutes) was similar to that of other useful PET neuroreceptor imaging agents.

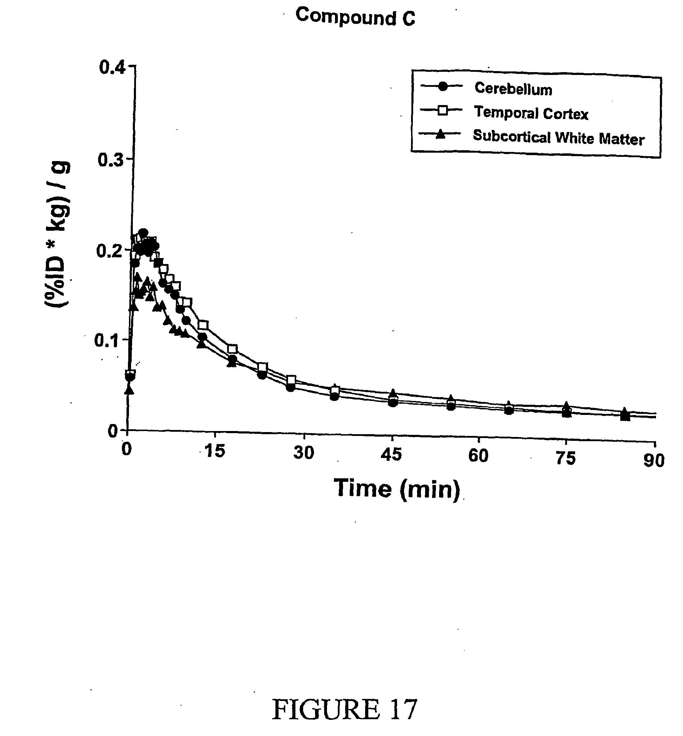

Figure 17 shows a representative PET TAC of radioactivity in three brain regions of a baboon following the intravenous injection of Compound C. The TACs indicate excellent brain penetration of radioactivity at early time points (about 0.22 % ID-kg/g, in good agreement to the brain penetration of Compound C in mice at 2 minutes post-injection) in all three regions and relatively rapid clearance of the regional radioactivity from 0-90 minutes post-injection in the brain of this control baboon. Regions of brain containing higher levels of white matter demonstrated slightly higher (< 10%) concentrations of radioactivity at 90 minutes than regions that were dominated by gray matter such as temporal cortex. The concentration of radioactivity in baboon cortex was nearly identical to that in the cerebellar cortex at all time points. The rate of clearance of radioactivity was considerably slower from baboon brain than from mouse brain, with Compound C exhibiting a clearance half-time of about 10 minutes from baboon brain gray matter. The radiotracer Compound C exhibited an early-to-late brain radioactivity concentration in baboon brain of about 6 indicating that only about 15% of the peak maximum radioactivity remained in brain at later time points. These results were consistent with the expected absence of amyloid plaques in the brains of these control animals and indicated that very little radioactivity was retained in normal baboon brain. Comparison of the in vivo behavior of Compound C in baboon brain to that of the entry and clearance of other successful PET radioligands in a reference brain region devoid of specific binding sites (i.e., cerebellum) was useful.

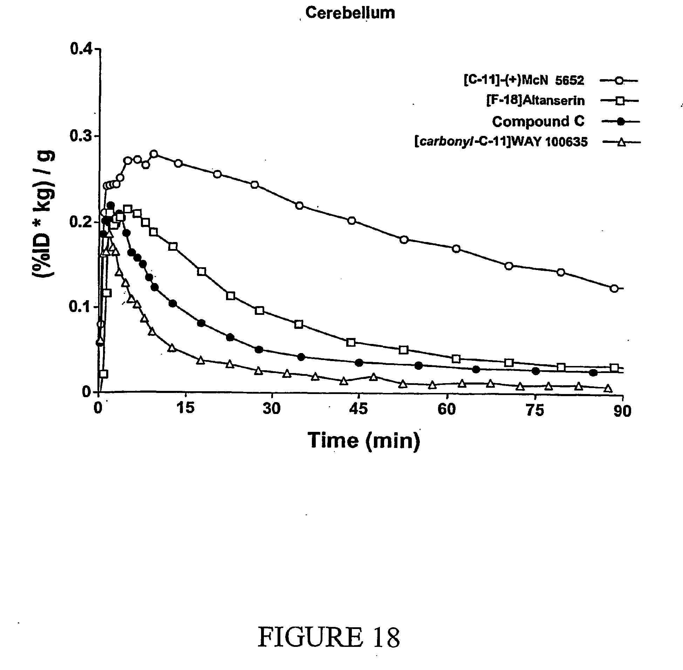

Figure 18 compares the cerebellar TACs in baboons of [carbonyl-¹¹C]WAY100635, [¹¹C](+)-McN5652, [¹⁸F]altanserin and Compound C. The relatively rapid non-specific binding clearance rates of [carbonyl-¹¹C]WAY100635 and [¹⁸F]altanserin are important to the success of these PET radioligands for imaging the serotonin 5-HT_{1A} and serotonin 5-HT_{2A} receptor systems. In contrast, the relatively slow in vivo clearance of [¹¹C](+)-McN5652 has limited the usefulness of this radioligand for imaging the serotonin transporter system. The brain clearance properties of Compound C indicated that the relatively rapid rate of non-specific clearance of this radiotracer (t_{1/2} =10 minutes) was similar to that of other useful PET neuroreceptor imaging agents.

## Claims

1. A compound of formula I 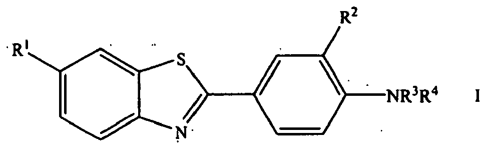 or a pharmaceutically acceptable salt, hydrate or solvate thereof, wherein:
R¹ is hydrogen, -OH, -NO₂, -CN, -COOR, -OCH₂OR, C₁-C₆ alkyl, C₂-C₆ alkenyl, C₂-C₆ alkynyl, C₁-C₆ alkoxy or halo, wherein one or more of the atoms of R¹ optionally is a radiolabeled atom;
R is C₁-C₆ alkyl, wherein one or more of the carbon atoms optionally is a radiolabeled atom;
R² is a radiofluoro;
R³ is hydrogen, C₁-C₆ alkyl, C₂-C₆ alkenyl or C₂-C₆ alkynyl; and
R⁴ is hydrogen-, C₁-C₆ alkyl, C₂-C₆ alkenyl or C₂-C₆ alkynyl.

2. The compound of claim 1, wherein:
R¹ is -OH or C₁-C₆ alkoxy;
R² is ¹⁸F; and
R³ and R4 are independently hydrogen or C₁-C₆ alkyl.

3. A compound of claim 2, having the following structure:

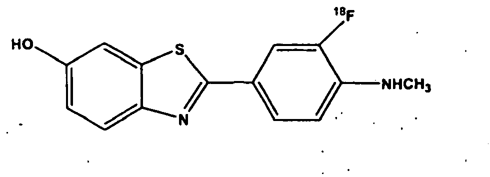

4. A pharmaceutical composition comprising
(i) an effective amount of a compound of any one of claims 1-3 and
(ii) a pharmaceutically acceptable carrier

5. Use of a compound according to any of claims 1-3 for the preparation of a composition for use in detecting amyloid deposits in vivo in a mammal.

## Patentansprüche

1. Verbindung der Formel I 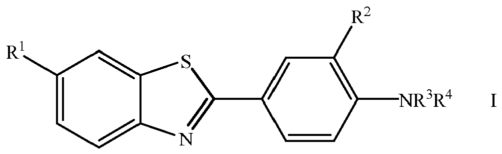 oder ein pharmazeutisch unbedenkliches Salz, Hydrat oder Solvat davon, worin:
R¹ Wasserstoff, -OH, -NO₂, -CN, -COOR, -OCH₂OR, C₁-C₆-Alkyl, C₂-C₆-Alkenyl, C₂-C₆-Alkinyl, C₁-C₆-Alkoxy oder Halogen ist, wobei eines oder mehrere der Atome von R¹ fakultativ ein radioaktiv markiertes Atom ist;
R C₁-C₆-Alkyl ist, wobei eines oder mehrere der Kohlenstofftome fakultativ ein radioaktiv markiertes Atom ist;
R² ein radioaktives Fluor ist;
R³ Wasserstoff, C₁-C₆-Alkyl, C₂-C₆-Alkenyl oder C₂-C₆-Alkinyl ist; und
R⁴ Wasserstoff, C₁-C₆-Alkyl, C₂-C₆-Alkenyl oder C₂-C₆-Alkinyl ist.

2. Verbindung nach Anspruch 1, worin
R¹ -OH oder C₁-C₆-Alkoxy ist;
R^{2 18}F ist; und
R³ und R⁴ unabhängig voneinander Wasserstoff oder C₁-C₆-Alkyl sind.

3. Verbindung nach Anspruch 2 mit der folgenden Struktur:

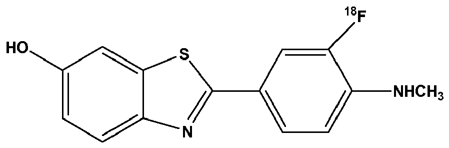

4. Pharmazeutische Zusammensetzung, umfassend:
(i) eine wirksame Menge einer Verbindung nach einem der Ansprüche 1-3 und
(ii) einen pharmazeutisch unbedenklichen Träger.

5. Verwendung einer Verbindung nach einem der Ansprüche 1-3 zur Herstellung einer Zusammensetzung zur Verwendung beim in vivo Nachweis von Amyloidablagerungen in einem Säugetier.

## Revendications

1. Composé de formule I 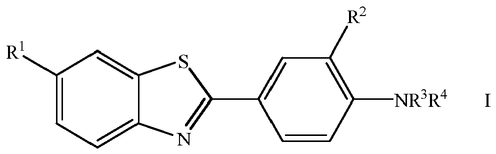 ou sel, hydrate ou solvate pharmaceutiquement acceptable de celui-ci, dans lequel :
R¹ est hydrogène, -OH, -NO₂, -CN, -COOR, -OCH₂OR, C₁-C₆ alkyle, C₂-C₆ alcényle, C₂-C₆ alcynyle, C₁-C₆ alkoxy ou halo, dans lequel un ou plusieurs des atomes de R¹ est éventuellement un atome radiomarqué ;
R est C₁-C₆ alkyle, dans lequel un ou plusieurs des atomes de carbone est éventuellement un atome radiomarqué ;
R² est un radiofluoro ;
R³ est hydrogène, C₁-C₆ alkyle, C₂-C₆ alcényle ou C₂-C₆ alcynyle ; et
R⁴ est hydrogène, C₁-C₆ alkyle, C₂-C₆ alcényle ou C₂-C₆ alcynyle.

2. Le composé de la revendication 1, dans lequel :
R¹ est -OH ou C₁-C₆ alkoxy ;
R² est ¹⁸F ; et
R³ et R⁴ sont indépendamment hydrogène ou C₁-C₆ alkyle.

3. Composé de la revendication 2, possédant la structure suivante :

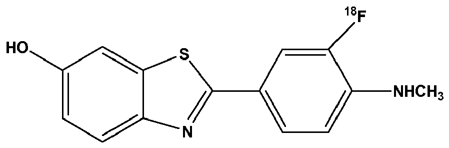

4. Composition pharmaceutique comprenant
(i) une quantité efficace d'un composé de l'une quelconque des revendications 1 à 3 et
(ii) un véhicule pharmaceutiquement acceptable.

5. Utilisation d'un composé selon l'une quelconque des revendications 1 à 3 pour la préparation d'une composition destinée à être utilisée pour détecter des dépôts amyloïdes *in vivo* chez un mammifère.
